(19) Europäisches Patentamt
European Patent Office
Office européen des brevets

(11) Veröffentlichungsnummer: **0 127 580**
**A2**

(12) # EUROPÄISCHE PATENTANMELDUNG

(21) Anmeldenummer: 84810253.9

(51) Int. Cl.³: A 61 K 7/06

(22) Anmeldetag: 24.05.84

(30) Priorität: 31.05.83 CH 2964/83

(43) Veröffentlichungstag der Anmeldung: 05.12.84 Patentblatt 84/49

(84) Benannte Vertragsstaaten: BE CH DE FR GB IT LI NL

(71) Anmelder: CIBA-GEIGY AG
Postfach
CH-4002 Basel(CH)

(72) Erfinder: Moldovanyi, Laszlo, Dr.
Austrasse 78
CH-4051 Basel(CH)

(72) Erfinder: Fearnley, Charles, Dr.
Wettsteinanlage 50
CH-4125 Riehen(CH)

(54) **Gemische aus quaternären, polymeren Ammoniumsalzen und einem ternären Tensidsystem aus nicht-ionogenen, anionischen und amphoteren Tensiden, deren Herstellung und Verwendung in kosmetischen Mitteln.**

(57) Wässrige Zusammensetzungen, die in neuartiger Kombination mindestens

(A) quaternäre, polymere Ammoniumsalze (Homo- oder Copolymere mit Molekulargewichten von $10^4$ bis $10^9$) und

(B) ein ternäres Tensidsystem aus

($B_1$) nicht-ionogenen tensiden,

($B_2$) anionischen Tensiden und

($B_3$) amphoteren tensiden

enthalten und als vollständig klare Formulierungen vorliegen, die durch Zusatz von Wasser als sogenannte Präzipitiersysteme ausfallen, sind als Haarkosmetika verwendbar, wobei bei dieser Applikationsart beim Shampoonieren ausgezeichnete Konditioniereffekte des behandelten Haars erzielt werden.

EP 0 127 580 A2

Croydon Printing Company Ltd

CIBA-GEIGY AG
Basel (Schweiz)

1-14447/+

Gemische aus quaternären, polymeren Ammoniumsalzen und einem ternären Tensidsystem aus nicht-ionogenen, anionischen und amphoteren Tensiden, deren Herstellung und Verwendung in kosmetischen Mitteln.

In der Haarpflege stellt die Reinigung der Haare einen der wichtigsten Vorgänge dar. Dabei werden als Reinigungsmittel zweckmässig synthetische Waschrohstoffe eingesetzt, die den Vorteil aufweisen, auch bei hoher Wasserhärte ihre volle Reinigungskraft zu behalten. Bei alleiniger Verwendung von Waschrohstoffen zeigt das behandelte Haar jedoch in der Regel keine Konditioniereffekte.

Der vorliegenden Erfindung liegt nun die Aufgabe zugrunde, ein für kosmetische Zwecke, insbesondere für die Haarpflege geeignetes kostengünstiges Reinigungsmittel zur Verfügung zu stellen, das neben einer guten Waschwirkung die Erzielung ausgezeichneter Konditioniereffekte auf dem Haar ermöglicht. Diese sog. Konditioniereffekte stellen einen Sammelbegriff für u.a. folgende erwünschte Eigenschaften des behandelten Haares dar:
- leichte Nass- und Trockenkämmbarkeit
- Verhinderung der statischen Aufladung
- keine negative Beeinträchtigung des ursprünglichen Glanzes
- Aspekt, insbesondere Volumen und Fülle
- Griff.

Aus den europäischen Patentanmeldungen 45 720 und 47 714 sind Zusammensetzungen aus polymeren quaternären Ammoniumsalzen und anionischen oder nicht-ionischen Tensiden bekannt, mit welchen gute Konditioniereffekte erreichbar sind. Diese bekannten Zusammensetzungen liegen in der Regel als opake bis cremeförmige Formulierungen vor.

In der Haarkosmetik besteht jedoch ein grosses Bedürfnis vor allem für klare, homogene Formulierungen, insbesondere Shampooformulierungen.

Beim Einsetzen von anionischen oder amphoteren Tensiden für sich allein neben polymeren quaternären Ammoniumsalzen können zwar auch klare Formulierungen erhalten werden, mit welchen jedoch unerwünschte, den Aspekt des behandelten Haars beeinträchtigende Akkumuliereffekte und/oder nur geringe Konditioniereffekte erreicht werden.

Es wurde nun gefunden, dass durch die Kombination von ausgewählten, polymeren, quaternären Ammoniumsalzen mit einem neuartigen, ternären Tensidsystem aus mindestens einem nicht-inogenen Tensid, mindestens einem anionischen Tensid und mindestens einem amphoteren Tensid klare, als Shampooformulierungen verwendbare Zusammensetzungen erhalten werden, worin das polymere, quaternäre Ammoniumsalz mit dem ternären Tensidsystem ein komplexes System bildet. Dieses komplexe System in den erfindungsgemässen Zusammensetzungen fällt auf Verdünnung mit Wasser aus und wird bei der Applikation als Shampooformulierung bei der Verschäumung auf dem Haar besonders gleichmässig verteilt. Dadurch sind auf unerwartete Weise Haarkonditioniereffekte erzielbar, die mindestens ebenbürtig oder sogar u.U. besser sind, als die Konditioniereffekte der bekannten, opaken bis cremeförmigen Zusammensetzungen.

Gegenstand der vorliegenden Erfindung ist somit eine als vollständig klare Formulierung vorliegende, wässrige Zusammensetzung aus polymeren Ammoniumsalzen und Tensiden, die dadurch gekennzeichnet ist, dass sie mindestens

(A) ein in wässrigen Tensidsystemen lösliches oder mikroemulgierbares, polymeres, quaternäres Ammoniumsalz, das eine Molekulargewichtsverteilung von $10^3$ bis $10^9$ aufweist, und

(B) ein ternäres Tensidsystem aus jeweils mindestens

($B_1$) einem nicht-ionogenen Tensid,

($B_2$) einem anionischen Tensid und

($B_3$) einem je eine positive und eine oder zwei negative Ladungen aufweisenden, amphoteren Tensid

enthält, wobei das Tensid als Komponente ($B_2$) und das amphotere Tensid, sofern es zwei negative Ladungen als Komponente ($B_3$) aufweist, mindestens teilweise mit der Komponente (A) unter Ionenaustausch umgesetzt ist.

Das Verfahren zur Herstellung der erfindungsgemässen Zusammensetzung und deren Verwendung in kosmetischen, vorzugsweise haarkosmetischen Mitteln bzw. das Haarbehandlungsverfahren, in welchem die erfindungsgemässe Zusammensetzung als haarkosmetisches Mittel verwendet wird, und die nach diesem Verfahren behandelten Haare die z.B. in Form von Perücken oder Toupets vorliegen, bilden weitere Gegenstände der vorliegenden Erfindung.

Bei den amphoteren Tensiden wird im Rahmen der vorliegenden Erfindung zwischen solchen mit überschüssigen, negativen Ladungen (zwitterionische Tenside mit vorzugsweise einer positiven Ladung und zwei negativen Ladungen) und solchen ohne überschüssige, negative Ladung (vorzugsweise mit intramolekular je einer negativen und positiven Ladung) unterschieden.

Als Komponente (A) der erfindungsgemässen Zusammensetzung kommen beispielsweise die folgenden, quaternären polymeren Ammoniumsalze in Betracht:

Quaternäre Stärke- oder Cellulose-Derivate, Poly(dialkylalkenylenammoniumhalogenid)-$\alpha,\omega$-(trialkanolammoniumhalogenid)-Verbindungen, Copolymerisate aus Dialkyldialkenylammoniumsalzen und Acrylamid oder Methacrylamid, Copolymerisate aus Acryl- oder Methacrylsäure-amid oder -nitril und quaternären Ammoniumsalzen der Acrylsäurereihe, insbesondere der Acryl- oder Methacrylsäureesterreihe oder der Acryl- oder Methacrylsäureamidreihe, oder Homopolymerisate aus Dialkyldialkenylammoniumsalzen oder Gemische der genannten polymeren Ammoniumsalze.

Bevorzugte, quaternäre Stärke- oder Cellulose-Derivate weisen als Komponente (A) ein Molekulargewicht von $10^3$ bis $2 \cdot 10^6$, vorzugsweise $10^4$ bis $10^6$, und Stärke- oder Cellulose-Einheiten auf, die in der Regel mit einem 1-Trimethylammonium-2-hydroxypropyl-Rest mindestens teilweise veräthert sind.

insbesondere weisen die Stärke- oder Cellulose-Einheiten solcher Derivate daher Reste der Formel

(1) 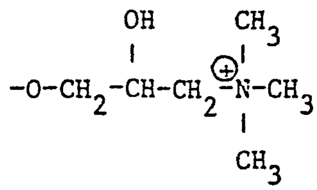 $Y_1^{\ominus}$ ,

worin $Y_1^{\ominus}$ das Anion einer Alkylcarbonsäure, vorzugsweise ein Alkylsulfat- oder Alkylphosphonatanion mit je 1 bis 4 Kohlenstoffatomen im Alkylrest oder insbesondere ein Halogenidanion bedeutet, und gegebenenfalls auch Reste der Formeln

(2) $-O-(CH_2-CH_2-O)_{m_1}-H$ ,

(3) 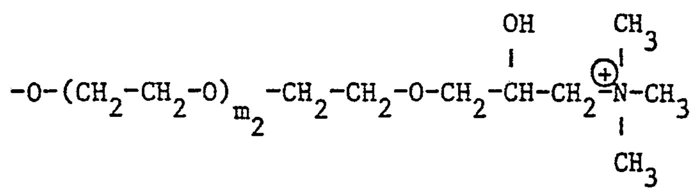 $Y_1^{\ominus}$

und

(4) 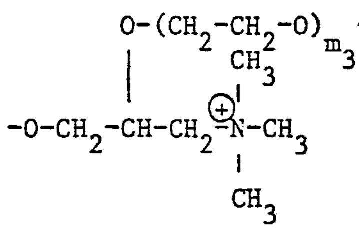 $Y_1^{\ominus}$

auf, worin $m_1$, $m_2$ und $m_3$ jeweils ganze Zahlen von 1 bis 50 bedeuten und $Y_1^{\ominus}$ die angegebenen Bedeutungen hat.

Cellulose-Derivate dieser Art sind z.B. in der französischen Patentschrift 1 492 597 beschrieben.

Bevorzugte Poly(dialkylalkenylenammoniumhalogenid)-α,ω-(trialkanolammoniumhalogenid)-Verbindungen als Komponente (A) sind z.B. Poly(dimethylbutenylenammoniumchlorid)-α,ω-bis(triäthanolammoniumchlorid)-Verbindungen, die ein Molekulargewicht von 1000 bis 5000 aufweisen und der Formel

(5) 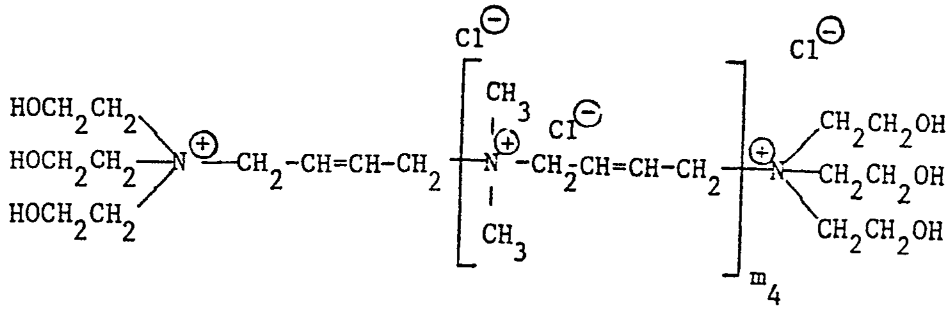

entsprechen, worin $m_4$ eine ganze Zahl von 4 bis 35 ist.

Bevorzugte Copolymerisate aus Dialkyldialkenylammoniumsalzen und Acrylamid oder Methacrylamid als Komponente (A) sind z.B. Copolymerisate aus Dimethyldiallylammoniumchlorid und Acrylamid, die ein Molekulargewicht von $5 \cdot 10^3$ bis $5 \cdot 10^6$, vorzugsweise $10^4$ bis $3 \cdot 10^6$ und in beliebiger Reihenfolge durchschnittlich 20 bis 99 Mol% wiederkehrende Strukturelemente der Formel

(6) 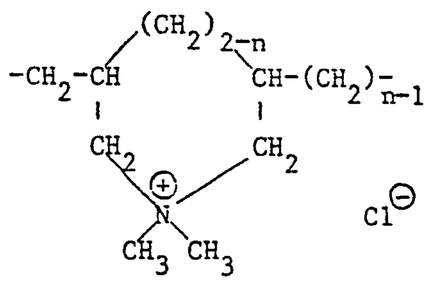 ,

worin n 1 oder 2 ist, und durchschnittlich 1 bis 80 Mol% wiederkehrende Strukturelemente der Formel

(7) 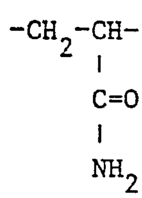

aufweisen. Bevorzugt sind Copolymerisate eines Molekulargewichtes von $10^4$ bis $3 \cdot 10^6$, welche in beliebiger Reihenfolge durchschnittlich 20 bis 90 Mol% wiederkehrende Strukturelemente der Formel (6), worin n 2 ist, und durchschnittlich 10 bis 80 Mol% wiederkehrende Strukturelemente der Formel (7) aufweisen.

Verbindungen der Formel (5) und Copolymerisate mit wiederkehrenden Strukturelementen der Formeln (6) und (7) sind z.B. in der deutschen Offenlegungsschrift 3 029 306 beschrieben.

Bevorzugte Copolymerisate aus Acryl- oder Methacrylsäure, und den entsprechenden Amiden und/oder Nitrilen und quaternären Ammoniumsalzen der Acrylsäurereihe als Komponente (A) weisen ein Molekulargewicht von $10^4$ bis $5.10^6$ auf. Solche Copolymerisate weisen wiederkehrende Strukturelemente der Formeln

(8) 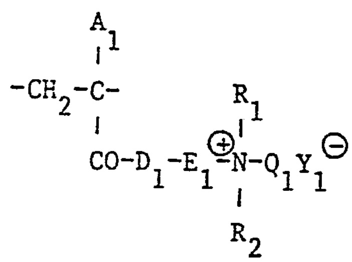 ,

(9) 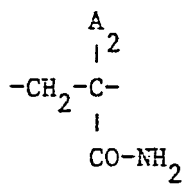

und gegebenenfalls

(10) 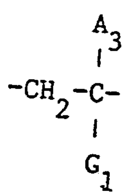 und

(11) 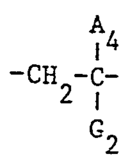

auf, worin $A_1$, $A_2$, $A_3$ und $A_4$ je Wasserstoff oder Methyl, $G_1$ und $G_2$ voneinander verschieden sind und je -CN, -COOH oder

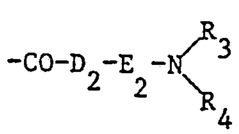 , $D_1$ und $D_2$ je Sauerstoff oder -NH-, $E_1$ und $E_2$ je

Alkylen mit 1 bis 4 Kohlenstoffatomen, das gegebenenfalls durch Hydroxyl substituiert ist, $R_1$, $R_2$, $R_3$ und $R_4$ je Methyl oder Aethyl, $Q_1$ Alkyl, Hydroxyalkyl mit 1 bis 4 Kohlenstoffatomen oder Benzyl bedeuten und $Y_1^{\ominus}$ die angegebenen Bedeutungen hat.

Die Copolymerisate der angegebenen Art können Strukturelemente der Formeln (8) und (9), der Formeln (8), (9) und (10) oder der Formeln (8), (9), (10) und (11) enthalten. Insbesondere weisen die Copolymerisate, sofern ihre Molekulargewicht $10^4$ bis $5.10^6$ beträgt, in beliebiger Reihenfolge durchschnittlich 1 bis 5 Mol% wiederkehrende Strukturelemente der Formel (8), durchschnittlich 75 bis 99 Mol% wiederkehrende Strukturelemente der Formel (9) und gegebenenfalls jeweils höchstens 10 Mol%, d.h. jeweils 0 bis durchschnittlich 10 Mol% wiederkehrende Strukturelemente der Formel (10) und/oder (11) auf.

In einer anderen, bevorzugten Ausführungsform, die im Vordergrund des Interesses steht, weisen solche Copolymere eine Molekulargewichtsverteilung von $10^4$ bis $10^9$ auf, wobei das Molekulargewicht von mindestens 5, vorzugsweise 5 bis 60, insbesondere 12 bis 50 Gewichtsprozent des Copolymers $10^7$ bis $19^9$ beträgt.

Solche Copolymerisate zeichnen sich dadurch aus, dass sie vorzugsweise durch Wasser-in-Oel Emulsionspolymerisation oder Lösungspolymerisation eines quaternären Ammoniumsalzes der Acrylsäurereihe und mindestens eines weiteren Comonomers auf Acrylbasis, erhältlich sind.

Durch Wasser-in-Oel Emulsionspolymerisation, auch inverse Emulsionspolymerisation genannt, oder durch Lösungspolymerisation, erreicht man den hohen Molekulargewichtsbereich der Copolymerisate von $10^7$ bis $10^9$, deren Anteil mindestens 5, vorzugsweise 10 bis 60 Gewichtsprozent der Copolymerisate innerhalb der breiten Molekulargewichts verteilung von $10^4$ bis $10^9$ ausmacht. Eine Anreicherung an Anteile eines Molekulargewichtes von $10^7$ bis $10^9$ innerhalb der breiten Molekulargewichtsverteilung von $10^4$ bis $10^9$ kann nötigenfalls auch durch Behandlung des Copolymers in einem vorzugsweise mit Wasser mischbaren Lösungsmittel, z.B. Methanol, Isopropanol oder

Aceton, erzielt werden. Eine solche Behandlung wird vor allem nach durchgeführter Lösungspolymerisation durchgeführt. Besonders bevorzugte Polymere weisen 12 bis 80, insbesondere 35 bis 45 Gewichtsprozent Anteile im Molekulargewichtsbereich von $10^7$ bis $10^9$ und weniger als 15 Gewichtsprozent Anteile im Molgewichtsbreich kleiner als $10^5$ auf.

Der Anteil an Strukturelementen der Formel (8) in den Copolymerisaten ist auch Quatgehalt genannt und bildet neben der Molekulargewichtsverteilung ein weiteres, wesentliches Kennzeichen der eingesetzten Ammoniumsalze, die mindestens 5 Gewichtsprozent Anteile im Molekulargewichtsbereich von $10^7$ bis $10^9$ enthalten, wobei durchschnittlich 5 bis 100, vorzugsweise 5 bis 80 und insbesondere 6 bis 40, bzw. 10 bis 30 Mol% Strukturelemente der Formel (8), 0 bis durchschnittlich 95, vorzugsweise 10 bis 95 oder 10 bis 75 und insbesondere 50 bis 90 Mol% Strukturelemente der Formel (9) und 0 bis durchschnittlich 10, vorzugsweise insgesamt 1 bis 8 Mol% Strukturelemente der Formel (10) und gegebenenfalls (11), d.h. (10) und/oder (11) und insbesondere je 1 bis 4 Mol% Strukturelemente der Formeln (10) und (11) vorhanden sind. Ammoniumsalze mit einem Quatgehalt, d.h. einem Gehalt an Strukturelementen der Formel (8) von 100%, stellen Homopolymerisate dar. Der Quatgehalt von 100% ist aber als Idealwert anzusehen, da durch Hydrolyse der Strukturelemente der Formel (8) die Homopolymerisate stets Spuren (z.B. 0,01 bis 0,5 Gewichtsprozent) an Strukturelementen der Formel (11) worin $G_2$ -COOH bedeutet, enthalten.

Copolymerisate, die Strukturelemente der Formeln (8), (9), (10) und (11), sind gegenüber Copolymerisaten, die Strukturlemente der Formeln (8), (9) und (10) aufweisen, bevorzugt. Im Vordergrund des Interesses stehen jedoch Copolymerisate, die nur die Strukturelemente der Formeln (8) und (9) aufweisen, wobei im allgemeinen durchschnittlich 1 bis 90, vorzugsweise 15 bis 80, insbesondere 20 bis 35 Mol % wiederkehrende Strukturelemente der Formel (8) und im allgemeinen durchschnittlich 10 bis 99, vorzugsweise 20 bis 85, insbesondere 65 bis 80 Mol % wiederkehrende Strukturelemente der Formel (9) vorhanden sind.

Als bevorzugte Bedeutungen für die Symbole der Formel (8) gelten für $A_1$ Methyl, für $D_1$ Sauerstoff, für $E_1$ unsubstituiertes n-Propylen oder insbesondere unsubstituiertes Aethylen, für $R_1$ und $R_2$ Methyl und für $Q_1$ unsubstituiertes Propyl, vorzugsweise Aethyl oder insbesondere Methyl. $A_2$ in Formel (9) steht vorzugsweise für Wasserstoff. Falls Strukturelemente der Formel (10) für sich allein, d.h. in Abwesenheit von Strukturelementen der Formel (11) vorhanden sind, gelten als bevorzugte Bedeutungen für die Symbole in Formel (10):

Methyl für $A_3$ und $-CO-D_2-E_2-$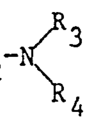 für $G_1$, wobei $D_2$, $E_2$, $R_3$ und $R_4$ die gleichen bevorzugten Bedeutungen haben wie für $D_1$, $E_1$, $R_1$ und $R_2$ vorstehend angegeben. Falls Strukturelemente der Formel (11) zusätzlich zu den Strukturelementen der Formel (10) vorhanden sind, bedeuten in Formel (11) $A_4$ vorzugsweise Wasserstoff und $G_2$ vorzugsweise -CN oder $A_4$ insbesondere Methyl und $G_2$ insbesondere -COOH.

Somit sind Copolymerisate im Vordergrund des Interesses, deren Molekulargewichtsverteilung $10^4$ bis $10^9$ beträgt, wobei etwa 10 bis 60 Gewichtsprozent der Copolymerisate ein Molekulargewicht von $10^7$ bis $10^9$ haben, und welche in beliebiger Reihenfolge durchschnittlich 1 bis 90 Mol% wiederkehrende Strukturelemente der Formel (8) und durchschnittlich 10 bis 99 Mol % wiederkehrende Strukturelemente der Formel (9) aufweisen und vor allem Copolymerisate, deren Molekulargewichtsverteilung $10^4$ bis $10^9$ beträgt, wobei 12 bis 50 Gewichtsprozent der Copolymerisate ein Molekulargewicht von $10^7$ bis $10^9$ hat, und welche in beliebiger Reihenfolge durchschnittlich 20 bis 35 Mol% wiederkehrende Strukturelemente der Formel

(12) 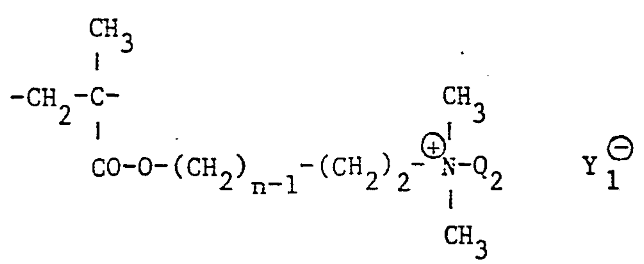 ,

worin n 1 oder 2 und $Q_2$ Propyl, Aethyl oder Methyl bedeuten und $Y_1^{\ominus}$ die angegebenen Bedeutungen hat,
und durchschnittlich 65 bis 80 Mol % wiederkehrende Strukturelemente der Formel (7) aufweisen.

Falls Homopolymerisate aus Dialkyldialkenylammoniumsalzen als Komponente (A) eingesetzt werden, so handelt es sich vorzugsweise um Dimethyldiallylammoniumchloride, die ein Molekulargewicht von 4000 bis 550 000 und wiederkehrende Strukturelemente der Formel (6) aufweisen und die ebenfalls in der deutschen Offenlegungsschrift 3 029 306 beschrieben sind.

Im Vordergrund des Interesses stehen als polymere Ammoniumsalze für die Komponente (A) der erfindungsgemässen Zusammensetzung Homopolymerisate, die wiederkehrende Strukturelemente der Formel (6), jedoch vorzugsweise Copolymerisate, die wiederkehrende Strukturelemente der Formel (5), oder der Formeln (6) und (7), vor allem der Formeln (8), (9) und gegebenenfalls (10) und (11) und insbesondere der Formeln (7) und (12) aufweisen.

Als nicht-ionogene Tenside für die Komponente ($B_1$) des ternären Tensidsystems (B) in den erfindungsgemässen Zusammensetzungen kommen alkoxylierte, vorzugsweise propoxylierte, insbesondere äthoxylierte Fettalkohole, Fettsäuren, Fettsäureamide, Alkylphenole oder Kohlenhydrate, Glycosidäther mit verätherten endständigen Hydroxylgruppen, die vorzugsweise als Alkenyl-, vor allem als Alkyläther mit 6 bis 14 Kohlenstoffatomen im Alkenyl- oder Alkylrest vorliegen, oder Addukte (Blockcopolymere) aus Aethylen- und Propylenoxyd in Betracht.

Geeignet sind ferner auch gegebenenfalls alkoxylierte, vorzugsweise propoxylierte, insbesondere äthoxylierte Fettsäureester von 3- bis 6-wertigen Alkoholen (Glycerin, Pentaerythrit, Sorbit oder insbesondere Sorbitan, d.h. cyclische Anhydrosorbite, erhalten aus Sorbit unter Abspaltung von Wasser) oder von Mono- und Disacchariden (Saccharose).

Die als nicht-ionogene Tenside verwendeten, äthoxylierten Fettalkohole, Fettsäuren, Fettsäureamide, Alkylphenole, Kohlenhydrate oder Kohlenhydratalkohole entsprechen vorzugsweise einer der Formeln

(13) $H-(O-CH_2-CH_2)_p-OOC-T_1$ ,

(14) $H-(O-CH_2-CH_2)_p-NH-CO-T_1$ ,

(15) $H-(O-CH_2-CH_2)_p-O-T_2$ ,

(16) $H-(O-CH_2-CH_2)_p-O-$ 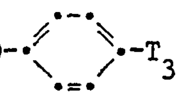 $-T_3$ oder

(17) $H-(O-CH_2-CH_2)_p-O-CH_2-(CHOH)_s-CH(H)_{n-1}-O(H)_{n-1}$ ,

worin $T_1$ Alkyl, durch Hydroxyl substituiertes oder vorzugsweise unsubstituiertes Alkenyl mit 7 bis 21, vorzugsweise 11 bis 17 Kohlenstoffatomen, $T_2$ Alkyl oder Alkenyl mit 8 bis 22, vorzugsweise 12 bis 18 Kohlenstoffatomen, $T_3$ Alkyl oder Alkenyl mit 6 bis 14, vorzugsweise 8 bis 12 Kohlenstoffatomen, n 2 oder vorzugsweise 1, p eine ganze Zahl von 1 bis 50, vorzugsweise 1 bis 20, insbesondere 9 bis 20 und s 3 oder vorzugsweise 4 bedeuten.

In Formel (13) leiten sich die Reste $T_1$-COO- von den entsprechenden gesättigten oder ungesättigten Fettsäuren mit 8 bis 22, vorzugsweise 12 bis 18 Kohlenstoffatomen ab.

Als Beispiel der entsprechenden Fettsäuren seien Capryl-, Caprin-, Arachin- und Behensäure, insbesondere Laurin-, Myristin-, Palmitin- und Stearinsäure oder Myristolein-, Palmitolein-, Elaeostearin-, Clupanodonsäure, insbesondere Oel-, Elaidin-, Eruka-, Ricinol-, Linol- und Linolensäure genannt. Alkyl- und Alkenylreste für $T_1$ die sich von technischen Gemischen der genannten gesättigten und/oder ungesättigten Fettsäuren ableiten, sind besonders bevorzugt.

Die Fettsäureamid- und Fettalkoholreste $T_1$-CO-NH- und $T_2$-O- in den Formeln (14) und (15) leiten sich vorzugsweise ebenfalls von den entsprechenden erwähnten Fettsäuren ab.

Bevorzugte Alkylreste für $T_3$ in Formel (16) sind z.B. Alkylreste mit 6 bis 12 Kohlenstoffatomen wie n-Hexyl, i-Hexyl, n-Heptyl, n-Octyl, i-Octyl, n-Nonyl, i-Nonyl, n-Decyl, n-Dodecyl, ferner Alkylreste mit 12 bis 14 Kohlenstoffatomen, wie Lauryl und Myristyl, Alkenylreste wie Oleyl und Reste, die sich von dimerisierten Olefinen mit je 6 oder 7 Kohlenstoffatomen ableiten.

Als äthoxylierte Kohlenhydrate kommen je nach dem Wert von s in Formel (17) vor allem äthoxylierte Pentosen und insbesondere Hexosen, z.B. äthoxylierte Glukose, in Betracht.

Unter den äthoxylierten, nicht-ionogenen Tensiden der Formeln (13) bis (17) sind die äthoxylierten Kohlenhydrate der Formel (17), vor allem die äthoxylierten Alkylphenole der Formel (16) und insbesondere die äthoxylierten Fettalkohole der Formel (15) bevorzugt. Als spezifisches Beispiel solcher äthoxylierter Alkylphenole sei u.a. das Tensid der Formel

(18) 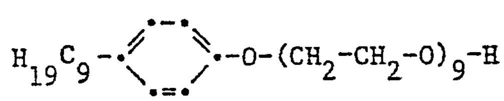

und als spezifische Beispiele solcher äthoxylierter Fettalkohole seien u.a. die Tenside der Formel

(19) $CH_3-(CH_2)_7-CH{=}CH-(CH_2)_8-O-(CH_2-CH_2-O)_{10}-H$ und

(20) $CH_3-(CH_2)_7-CH{=}CH-(CH_2)_8-O-(CH_2-CH_2-O)_{20}-H$,

wobei das Tensid der Formel (19) und vor allem der Formel (20) im Vordergrund des Interesses steht.

Bei einer weiteren, ebenfalls bevorzugten Ausführungsform der nicht-ionogenen Tenside handelt es sich um Glykosidalkenyläther oder -alkyläther der Formel

(21) $$T_3\text{-O-}CH_2\text{-(CHOH)}_3\text{-CH-}CH_2OH,$$
$$\lfloor_{2}\text{———}O\text{—}_{3}\rfloor$$

worin $T_3$ die angegebene Bedeutung hat. Als spezifisches Beispiel solcher Glycosidäther sei u.a. das Tensid der Formel

(22) $$H_3C\text{-}(CH_2)_{7-9}\text{-O-CH-(CHOH)}_3\text{-CH-}CH_2OH$$
$$\lfloor\text{———}O\text{—}_{3}\rfloor$$

genannt, das ebenfalls im Vordergrund des Interesses steht.

Als nicht-ionogene Tenside sind ebenfalls handelsübliche Produkte aus Aethylenoxyd und Propylenoxyd bevorzugt, die durch Addition von Aethylenoxyd an ein Kondensationsprodukt aus Propylenoxd mit Propylenglykol erhältlich sind und Molekulargewichte von etwa 1000 bis etwa 15000 auf weisen. Solche Addukte stellen Blockcopolymerisate dar, die der wahrscheinlichen Formel

(23) $$HO\text{-}(CH_2\text{-}CH_2\text{-}O)_x\text{-}(CH_2\text{-}\underset{\displaystyle CH_3}{\underset{|}{CH}}\text{-}O)_y\text{-}(CH_2\text{-}CH_2\text{-}O)_z\text{-}H$$

entsprechen, worin x, y und z gleiche oder voneinander verschiedene, ganze Zahlen bedeuten. Die Werte für x, y und z hängen vom Molekulargewicht des Copolymerisates ab und stellen nur Durchschnittswerte dar. Besonders bevorzugt sind Copolymerisate mit durchschnittlichen Molekulargewichten von 2000 bis 8000, worin die Durchschnittswerte von x und z je zwischen 2 und 60 und von y zwischen 20 und 80 schwanken und somit der wahrscheinlichen Formel

(24) $$HO\text{-}(CH_2\text{-}CH_2\text{-}O)_{2-60}\text{-}(CH_2\text{-}\underset{\displaystyle CH_3}{\underset{|}{CH}}\text{-}O)_{20-80}\text{-}(CH_2\text{-}CH_2\text{-}O)_{2-60}\text{-}H$$

entsprechen. Nicht-ionogene Tenside der wahrscheinlichen Formel (24) stehen ebenfalls im Vordergrund des Interesses.

Als weitere, bevorzugte Ausführungsform von nicht-ionogenen Tensiden kommen ebenfalls Addukte der Blockpolymerisate der Formeln (23) und (24) in Betracht, die zusätzlich an Aethylendiamin angelagert sind, d.h. Addukte aus Aethylenoxyd, Propylenoxyd und Aethylendiamin.

Bei den als nicht-ionogene Tenside ebenfalls in Betracht kommenden Fettsäureestern von 3- bis 6-wertigen Alkoholen, die gegebenenfalls alkoxyliert, vorzugsweise propoxyliert oder äthoxyliert sind, insbesondere Fettsäureestern von Sorbitan, handelt es sich vorzugsweise um Monoester der Formel

(25) 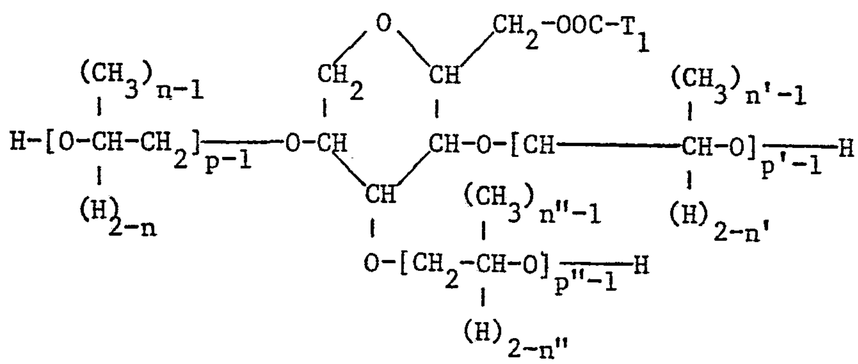 oder

(26) 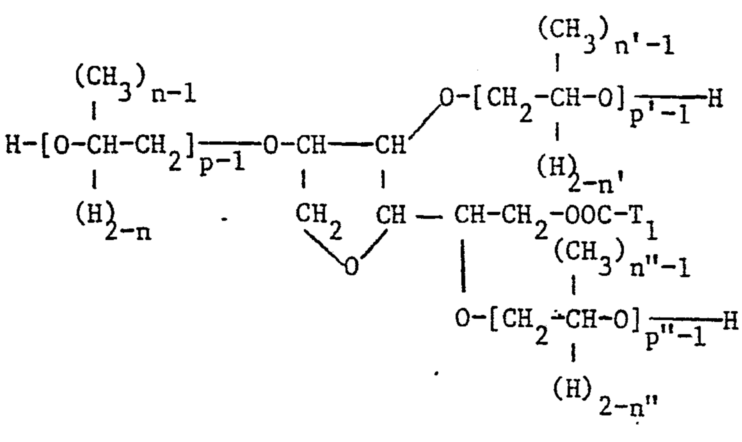

worin $T_1$ die angegebenen Bedeutungen hat, n, n' voneinander verschieden oder vorzugsweise gleich sind und jeweils für die Zahl 1 oder 2 stehen, p, p' und p" voneinander verschieden oder vorzugsweise gleich sind und p' und p" die für p angegebene Bedeutung haben, d.h. für eine ganze Zahl von 1 bis 50, vorzugsweise 1 bis 20 und insbesondere 9 bis 20 stehen. Sofern p, p' und p" in Formeln (25) und (26) $>1$ sind,

werden die entsprechenden Verbindungen aus einem Alkylenoxyd hergestellt, nämlich aus Propylenoxyd falls n, n' und n" für 2 stehen oder vorzugsweise aus Aethylenoxyd, falls n, n' und n" für 1 stehen.

Als spezifische Vertreter solcher nicht-ionischer Tenside seien die gegebenenfalls äthoxylierten Monoester der Formel

(27)

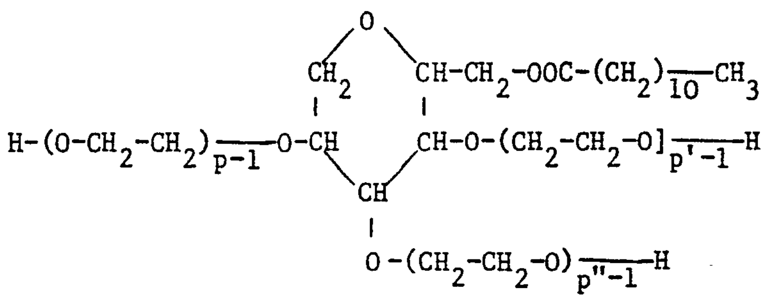

oder

(28)

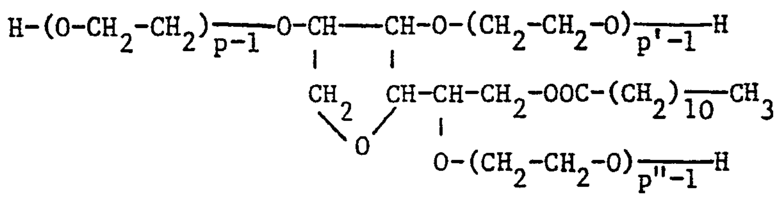

genannt, worin p, p' und p" die angegebenen Bedeutungen haben.

Auch Gemische nicht-ionogener Tenside der angegebenen Art, z.B. solche der Formel (13) bis (24), vor allem (15), (16), (17), (18), (21), (23), (25) und (26), insbesondere (19), (20), (22), (24), (27) und (28) kommen als Komponente ($B_1$) des ternären Tensidsystems (B) in den erfindungsgemässen Zusammensetzungen in Frage.

Anionische Tenside, die als Komponente ($B_2$) des ternären Tensid-Systems (B) in den erfindungsgemässen Zusammensetzungen enthalten sind, entsprechen der Formel

(29) $X_1^{\ominus} Z^{\oplus}$ ,

worin $Z^{\oplus}$ ein Alkalimetall-, insbesondere Natrium-, oder ein Ammoniumkation bedeutet, wobei das Ammoniumkation ($NH_4^{\oplus}$) unsubstituiert oder vorzugsweise durch 1, 2 oder vor allem 3 Alkyl- oder Alkanolreste mit je 1 bis 4 Kohlenstoffatomen substituiert ist und $X_1^{\ominus}$ für den Rest eines oberflächenaktiven Sarkosinats, Sulfates, (z.B. Alkylsulfates, Alkyläthersulfates, Alkylamidsulfates, Alkylamidäthersulfates, Alkylarylpolyäthersulfates oder Monoglyceridsulfates) Sulfonates (z.B. Alkylsulfonates, Alkylamidsulfonates, Alkylarylsulfonates, α-Olefinsulfonates) oder Sulfobernsteinsäurederivates (z.B. Alkylsulfosuccinats, Alkyläthersulfosuccinats, Alkylamidsulfosuccinats, Alkylamidpolyäthersulfosuccinats oder Alkylsulfosuccinamids) steht.

In Frage kommen z.B. für Sarkosinat-Tenside solche der Formel

(30) 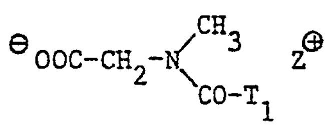 ,

worin $T_1$ und $Z^{\oplus}$ die angegebenen Bedeutungen haben.

In Formel (26) leiten sich die Reste $T_1CO-$ von den entsprechenden gesättigten oder ungesättigten Fettsäuren mit 8 bis 22, vorzugsweise 12 bis 18 Kohlenstoffatomen ab. Hierbei handelt es sich im allgemeinen um die bei der Definition des Restes $T_1COO-$ in Formel (13) vorstehend erwähnten Fettsäuren bzw. Fettsäure-Gemische. Als spezifische Vertreter von Sarkosinat-Tensiden seien vor allem solche der Formel

(31) 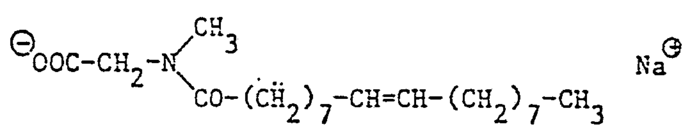

und insbesondere der Formel

(32) 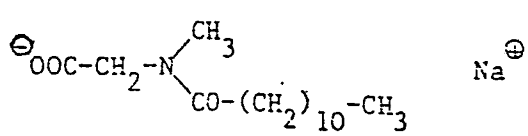

genannt.

Falls $X_1^{\ominus}$ einen Sulfatrest (von Alkylsulfaten, sowie von Aether-, Ester-, Amid- oder Aminosulfaten) bedeutet, kommen z.B. Tenside der Formeln

(33) $T_1-O-(CH_2-CH_2-O)_{p-1}-SO_3^{\ominus}Z^{\oplus}$,

(34) 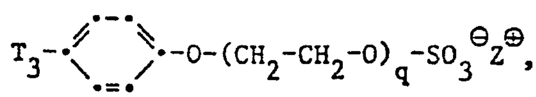,

(35) $T_1-CO-NH-(CH_2)_r-O-SO_3^{\ominus}Z^{\oplus}$ oder

(36) $T_1-CO-NH-(CH_2-CH_2-O)_p-SO_3^{\ominus}Z^{\oplus}$

in Betracht, worin $T_1$, $T_3$, $Z^{\oplus}$ und p die angegebenen Bedeutungen haben und q eine ganze Zahl von 6 bis 12 und r eine ganze Zahl von 2 bis 6 bedeuten.

Als spezifische Vertreter von Sulfat-Tensiden seien vor allem solche der Formeln

(37) $^{\ominus}O_3S-O-(CH_2)_{2-4}-NH-CO-(CH_2)_{11}-CH_3\ Na^{\oplus}$,

(38) $^{\ominus}O_3S-(O-CH_2-CH_2)_{8-10}-NH-CO-(CH_2)_{11}-CH_3\ Na^{\oplus}$ und

(39) 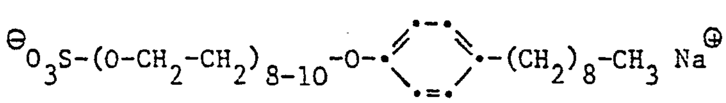

und insbesondere der Formeln

(40) $^{\ominus}O_3S-(O-CH_2-CH_2)_2-O-(CH_2)_{11}-CH_3\ Na^{\oplus}$ und

(41) 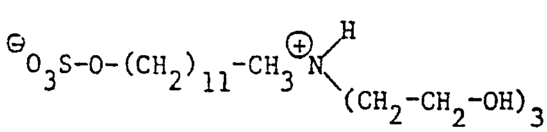

genannt.

Ist $X_1^{\ominus}$ ein Sulfonatrest, so kommen z.B. Tenside einer der Formeln

(42) 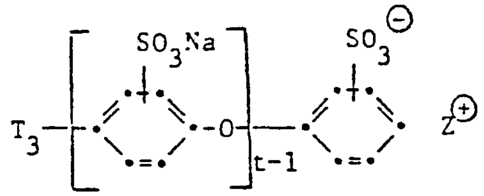 ,

(43)
$SO_3^{\ominus}$
$T_3-CH-(CH_2)_r-CH_3 \quad Z^{\oplus}$ ,

(44) $T_1-CH_2-SO_3^{\ominus} Z^{\oplus}$ ,

(45) $T_3-COO-CH_2-CH_2-SO_3^{\ominus} \quad Z^{\oplus}$ ,

(46)
$CH_3$
$T_3-CO-N-CH_2-CH_2-SO_3^{\ominus} \quad Z^{\oplus}$ ,

(47)
$SO_3$
$T_1-CH_2-CH-T_1' \quad Z^{\oplus}$ ,

(48) $T_1-CH=CH-SO_3^{\ominus} \quad Z^{\oplus}$ oder

(49)
OH
$T_1-CH-CH_2-SO_3^{\ominus} \quad Z^{\oplus}$

in Betracht, worin $T_1$, $T_3$, $Z^{\oplus}$ und r die angegebenen Bedeutungen haben, $T_1'$ die für $T_1$ angegebenen Bedeutungen hat, wobei $T_1$ und $T_1'$ gleich oder voneinander verschieden sind und t 1 oder 2 bedeutet.

Als spezifische Vertreter von Sulfonat-Tensiden seien vor allem solche der Formeln

(50)
$SO_3^{\ominus}$
$CH_3-(CH_2)_{11}-CH-(CH_2)_{2-4}-CH_3 \; Na^{\oplus}$ ,

(51) $^{\ominus}O_3S-(CH_2)_2-OOC-(CH_2)_{10}-CH_3 \quad NH_4^{\oplus}$ und

(52) 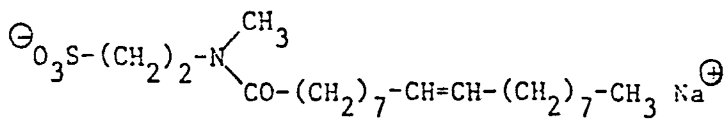 ,

vorzugsweise der Formel

(53) 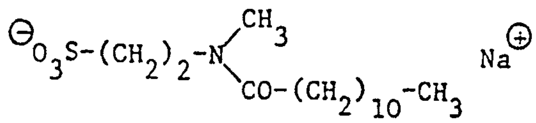 und

(54) 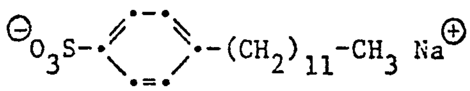

und insbesondere der Formel

(55) $CH_3-(CH_2)_{13-17}-SO_3^{\ominus}$ $Na^{\oplus}$

erwähnt.

Bedeutet $X_1^{\ominus}$ den Rest eines Sulfobernsteinsäurederivats, so entspricht das Tensid z.B. einer der Formeln

(56) 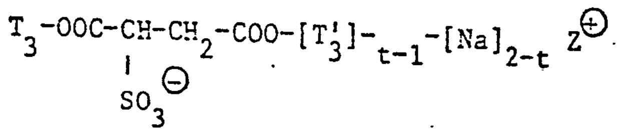 ,

(57) 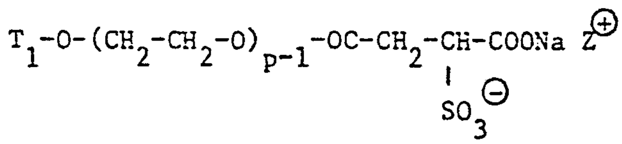 ,

(58) 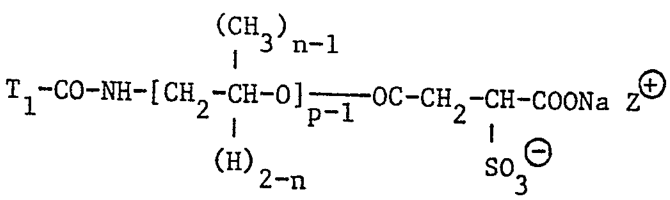 ,

(59) 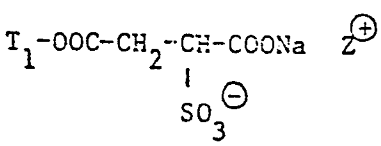 ,

(60) 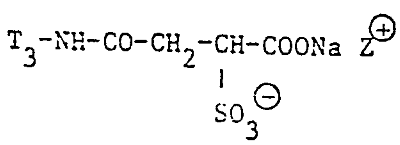 ,

(61) 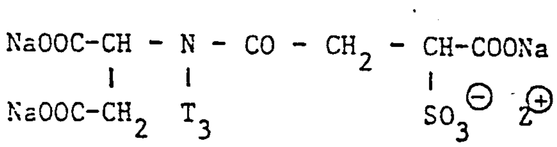 oder

(62) 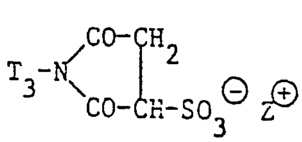

worin n 1 oder 2 ist, $T_1$, $T_3$, $Z^{\oplus}$, p und t die angegebenen Bedeutungen haben und $T'_3$ die für $T_3$ angegebenen Bedeutungen hat, wobei $T_3$ und $T'_3$ gleich oder voneinander verschieden sind.

Als spezifische Vertreter solcher Tenside seien die Sulfobernsteinsäurederivate der Formeln

(63) 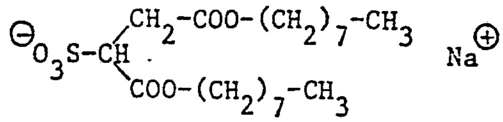 ,

(64) 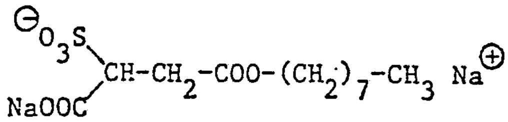 ,

(65) 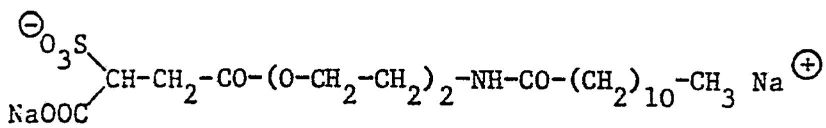 ,

(66) 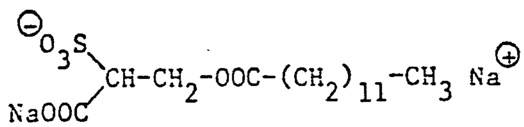 ,

(67) 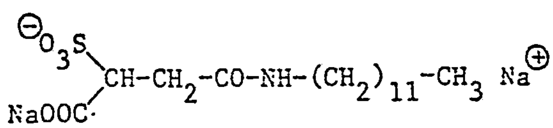 ,

(68) 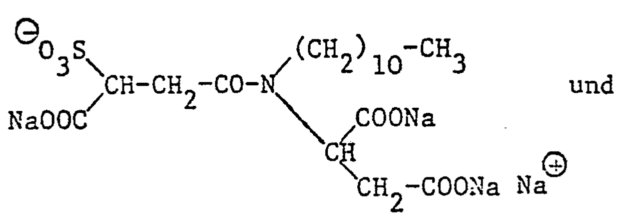 und

(69) 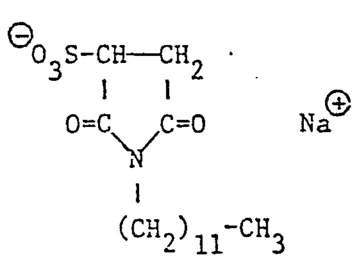 , vorzugsweise der Formel

(70) $$\begin{matrix} ^{\ominus}O_3S \\ NaOOC \end{matrix}\!\!>CH-CH_2-COO-(CH_2-CH_2-O)_2-(CH_2)_{11}-CH_3 \quad Na^{\oplus}$$

erwähnt.

Im Vordergrund des Interesses stehen als Komponente ($B_2$) das anionische Sulfonat-Tensid der Formel (55) und vor allem das anionische Sarkosinat-Tensid der Formel (32) und die anionischen Sulfat-Tenside der Formeln (40) und (41). Auch Gemische anionischer Tenside der angegebenen Art, z.B. solche der Formeln (30) bis (70), insbesondere der Formeln (32), (40), (41) und (55) kommen als Komponente ($B_2$) in Frage.

Bei den amphoteren Tensiden als Komponente ($B_3$) des ternären Tensid-Systems (B) der erfindungsgemässen Zusammensetzungen handelt es sich entweder um Tenside ohne überschüssige negative Ladungen, die vorzugsweise intramolekular je eine negative und positive Ladung aufweisen, vor allem um Betaine oder Sulfobetaine, die sich von aliphatischen Aminen ableiten, oder um zwitterionische Tenside mit überschüssigen negativen Ladungen, die vorzugsweise zwei negative Ladungen und eine positive Ladung aufweisen, vor allem um Tenside der Formel

(71) $$X_2^{\ominus} Z^{\oplus} ,$$

worin $X_2$ den zwei negative Ladungen aufweisenden Rest eines oberflächenaktiven N-Alkyl-iminodipropionats und insbedere eines Dicarbonsäurederivates, das einen Glykokollrest enthält, bedeutet.

Die als Komponente ($B_3$) erfindungsgemäss eingesetzten amphoteren Tenside ohne überschüssige negative Ladungen, die Betainderivate darstellen, welche sich von einem aliphatischen Amin ableiten, entsprechen vorzugsweise einer der Formeln

(72) 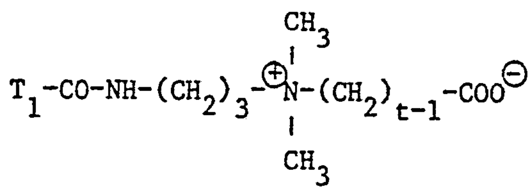 ,

(73) 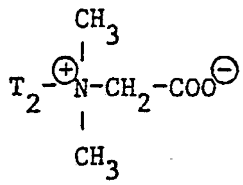 ,

(74) $T_2-\overset{\oplus}{N}H_2-CH_2-(CH_2)_{t-1}-COO^{\ominus}$ ,

(75) $T_2-(NH-CH_2-CH_2)_{t-1}-NH-CH_2-CH_2-\overset{\oplus}{N}H_2-CH_2-COO^{\ominus}$ ,

(76) 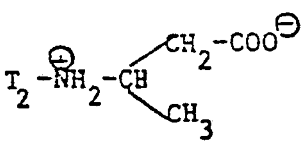 ,

(77) 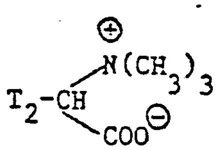 oder

(78) 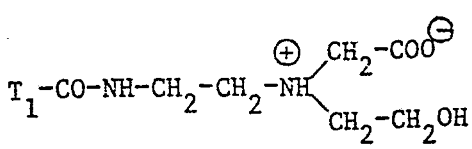 ,

worin $T_1$, $T_2$ und t die angegebenen Bedeutungen haben.

Als Beispiele spezifischer Vertreter solcher Betainderivate seien u.a. die Tenside bzw. technischen Tensidgemische der Formeln

(79) 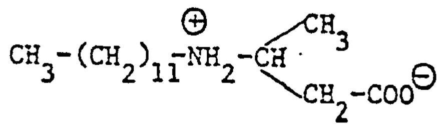 und

(80) 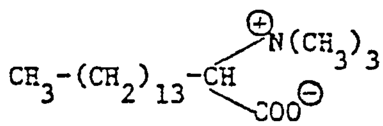 ,

vorzugsweise der Formeln

(81) 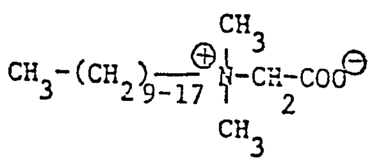 und

(82) 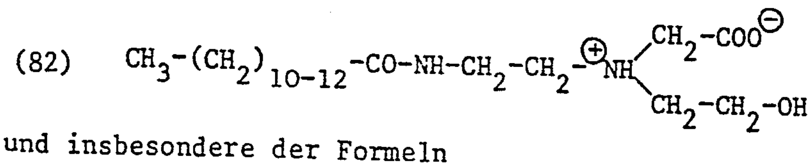

und insbesondere der Formeln

(83) $CH_3-(CH_2)_{11-13}-\overset{\oplus}{N}H_2-(CH_2)_2-COO^{\ominus}$ und

(84) 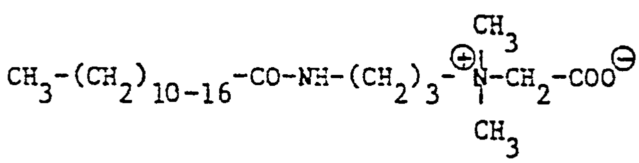

genannt.

Die als Komponentete ($B_3$) erfindungsgemäss eingesetzten amphoteren Tenside ohne überschüssige negative Ladungen, die Sulfobetainderivate darstellen, die sich von einem aliphatischen Amin ableiten, entsprechen vorzugsweise einer der Formeln

(85) 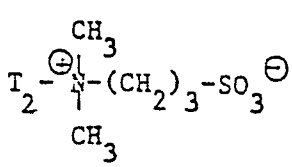 ,

(86) $T_2-\overset{\oplus}{N}H_2-(CH_2)_2-SO_3^{\ominus}$

(87) 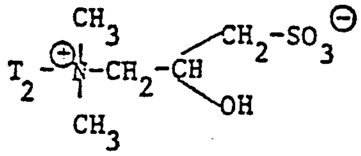 ,

(88) 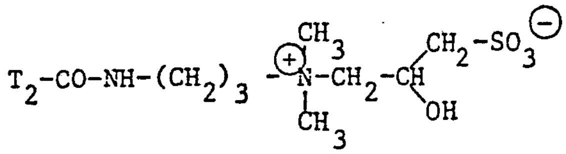

(89) 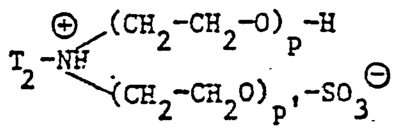

(90) 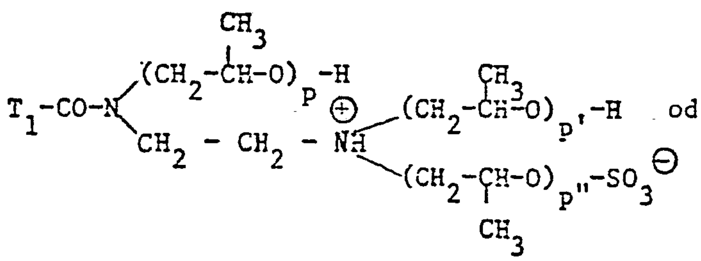 oder

(91) 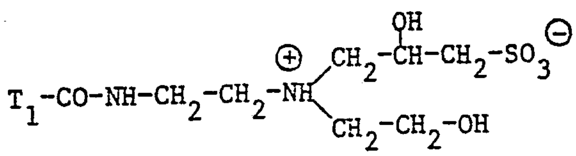 ,

worin $T_1$ und $T_2$ die angegebenen Bedeutungen und p' und p" die für p angegebenen Bedeutungen haben, wobei p, p' und p" gleich oder voneinander verschieden sind.

Als Beispiele spezifischer Vertreter solcher Sulfobetainderivate seien u.a. die Tenside der Formeln

(92) 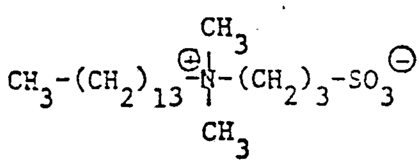 ,

(93) 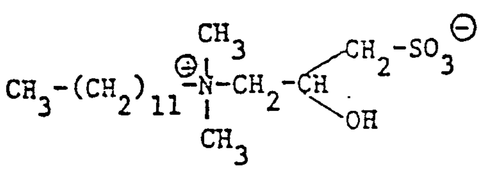 und

(94) 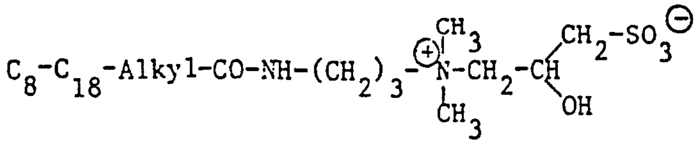

($C_8$-$C_{18}$ = technisches Gemisch aus Kokosfettsäure aus
ca. 15% $C_8$- und $C_{10}$-Alkyl
ca. 40% $C_{12}$-Alkyl
ca. 30% $C_{14}$-Alkyl und
ca. 15% $C_{16}$- und $C_{18}$-Alkyl)

und vorzugsweise der Formeln

(95) $CH_3-(CH_2)_{11-13}-\overset{\oplus}{N}H_2-(CH_2)_2-SO_3^{\ominus}$ und

(96) 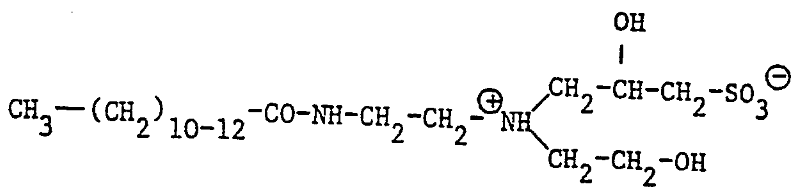

genannt.

Die als Komponente ($B_3$) eingesetzten amphoteren Tenside mit überschüssigen negativen Ladungen der Formel (71), worin $X_2$ den Rest eines oberflächenaktiven N-Alkyl-iminodipropionats bedeutet, entsprechen vorzugsweise der Formel

(97) 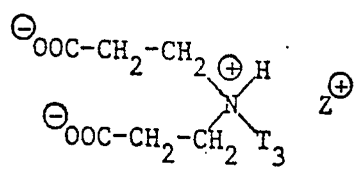 ,

worin $T_3$ und $Z^{\oplus}$ die angegebenen Bedeutungen haben.

Als spezifische Beispiele seien die Tenside der Formeln

(98) 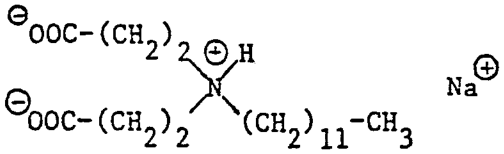

und

(99) 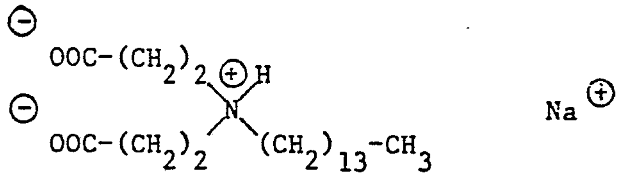

erwähnt.

Amphotere Tenside ($B_3$) mit überschüssigen Ladungen der Formel (71), worin $X_2$ den Rest eines Glykokollrest enthaltenden Dicarbonsäurederivats bedeutet, entsprechen vorzugsweise der Formel

(100) 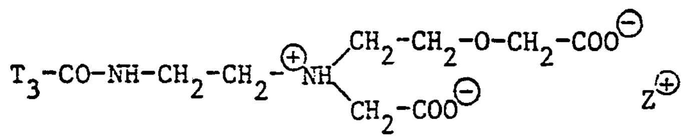 ,

worin $T_3$ und $Z^{\oplus}$ die angegebenen Bedeutungen haben.

Als spezifische Vertreter seien u.a. die Tenside der Formel

(101) 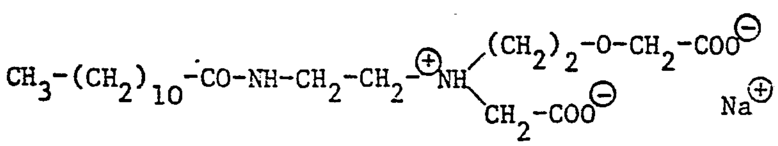 und

(102) 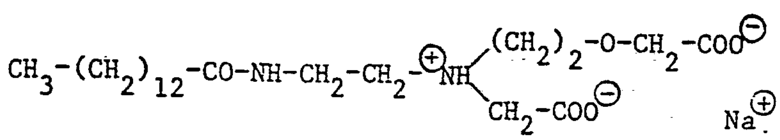

erwähnt.

In einer besonderen Ausführungsart liegen die Betainderivate oder Sulfobetainderivate der Formeln (78) und (91) und die einen Glykokollrest aufweisenden Dicarbonsäurederivate der Formel (100), bzw. deren bevorzugte Vertreter der Formeln (82), (96) und (97) gegebenenfalls mindestens teilweise als cyclische Imidazoliniumderivate vor, sofern der pH-Wert der wässrigen Lösungen der entsprechenden Tenside im alkalischen Bereich liegt, d.h. über etwa 7 beträgt.

Im alkalischen Medium entsprechen somit solche Imidazoliniumderivate mindestens teilweise einer der Formeln

(103) 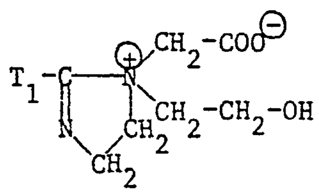 ,

(104) 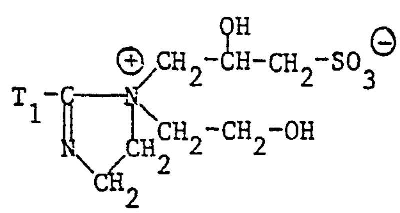 oder

(105) 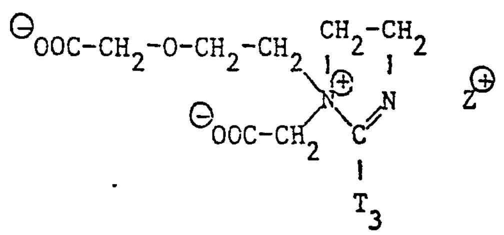 ,

worin $T_1$, $T_3$ und $Z^{\oplus}$ die angegebenen Bedeutungen haben.

Als Beispiele spezifischer Vertreter solcher Imidazoliniumbetaine oder -sulfobetaine oder solcher in 2-Stellung alkylsubstituierten Imidazolinium-Dicarbonsäurederivate seien u.a. die Tenside, bzw. technischen Tensidgemische der Formeln

(106) 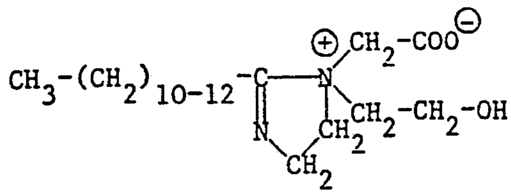 ,

(107) 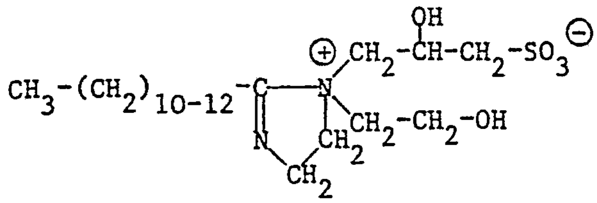 ,

(108) 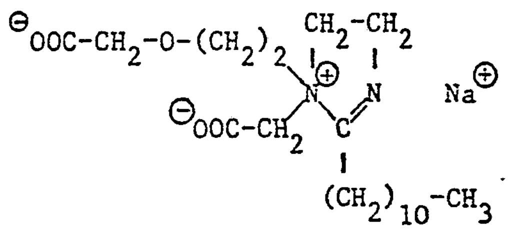

und

(109) 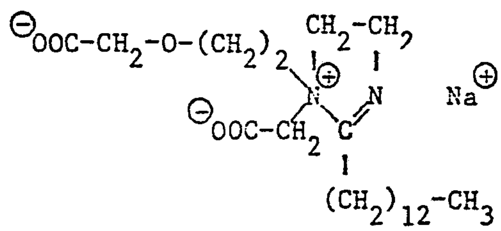

erwähnt.

Im Vordergrund des Interesses stehen als Komponente ($B_3$) die amphoteren Tenside der Formeln (81), (82) bzw. (106), (95), (96) bzw. (107), vor allem der Formeln (101) und (102), bzw. (108) und (109) und insbesondere der Formeln (83) und (84).

Auch gemischte amphotere Tenside der angegebenen Art, d.h. solche der Formel (72) bis (109), vor allem der Formeln (81), (82), (95), (96), (101), (102), (107), (108) und (109) und insbesondere der Formeln (83) und (84) kommen in Frage.

In bevorzugter Kombination enthalten die erfindungsgemässen Zusammensetzungen, die im Vordergrund des Interesses stehen,

(A) ein Poly(dimethylbutenylammoniumchlorid)-α,ω-bis-(triäthanolammoniumchlorid) eines Molekulargewichtes von 1000 bis 5000 der Formel (5),

vor allem ein Homopolymerisat aus Dimethyldiallylammoniumchlorid eines Molekulargewichts von 4000 bis 550 000 mit wiederkehrenden Strukturelementen der Formel (6), worin n 2 ist, oder eines entsprechenden Mischpolymerisats, worin n in Formel (6) alternierend für 1 und für 2 steht,

insbesondere ein Copolymerisat aus Acrylamid und quaternären Ammoniumsalzen der Acrylsäurereihe einer Molekulargewichtsverteilung von $10^4$ bis $10^9$, wobei das Molekulargewicht von etwa 12 bis etwa 50 Gewichtsprozent des Copolymerisats $10^7$ bis $10^9$ beträgt, mit durchschnittlich 65 bis 80 Mol % wiederkehrenden Strukturelementen der Formel (7) und durchschnittlich 20 bis 35 Mol % wiederkehrenden Strukturelementen der Formel (12),

als polymeres, quaternäres Ammoniumsalz,

($B_1$) den Glycosidäther der Formel (22), vor allem das Addukt aus Aethylenoxyd und Propylenoxyd eines Molekulargewichtes von 2000 bis 8000 der Formel (24), den äthoxylierten Fettalkohol der Formel (19) oder insbesondere (20) oder den äthoxylierten Sorbitan-Fettsäuremonoester der Formel (27) oder (28) als nicht-ionogenes Tensid,

($B_2$) den Alkylsulfonat der Formel (55), vor allem den Sarkosinat der Formel (32) und die Sulfate der Formel (40) oder (41) als anionisches Tensid und

($B_3$) das einen Glykolrest aufweisende Dicarbonsäurederivat der Formel (101) oder (102) bzw. das Imidazoliniumderivat der Formel (108) oder (109), vor allem das Betainderivat der Formel (83) oder insbesondere (84) als amphoteres Tensid.

Bevorzugte, erfindungsgemässe Zusammensetzungen enthalten 0,05 bis 2, vorzugsweise 0,1 bis 0,8, insbesondere 0,1 bis 0,5 Gewichtsprozent, bezogen auf Wirksubstanz (d.h. wasserfreie Substanz), des Ammoniumsalzes als Komponente (A) und 4 bis 40, vorzugsweise 7 bis 75, insbesondere 15 bis 25 Gewichtsprozent, bezogen auf Wirksubstanz des ternären Tensidsystems als Komponente (B). Das Tensidsystem (B) enthält seinerseits das nichtionogene Tensid ($B_1$), das anionische Tensid ($B_2$) und das amphotere Tensid ($B_3$) in einem Gewichtsverhältnis ($B_1$):($B_2$):($B_3$) von 1:(0,02 bis 6,0):(0,2 bis 4,0), vorzugsweise von 1:(0,04 bis 1,5):(0,3 bis 3,3), insbesondere 1:(0,1 bis 1,8):(1,0 bis 1,8).

Im Vordergrund des Interesses stehen erfindungsgemässe Zusammensetzungen, die

0,1 bis 0,7, vorzugsweise 0,1 bis 0,5 Gewichtsprozent der Komponente (A),
1 bis 10 Gewichtsprozent der Komponente ($B_1$),
0,1 bis 15, vorzugsweise 0,4 bis 10,0 Gewichtsprozent der Komponente ($B_2$) und
1 bis 15, vorzugsweise 1,2 bis 10,0 Gewichtsprozent der Komponente ($B_3$),

bezogen auf Wirksubstanz, enthalten.

In der Regel weisen die Zusammensetzungen einen pH-Wert von 4 bis 8, vorzugsweise 5 bis 7, insbesondere 5 bis 6 auf.

Zusätzlich zu den Komponenten (A), ($B_1$), ($B_2$) und ($B_3$) können die Zusammensetzungen kosmetische Hilfsmittel als fakultative Komponente (C) enthalten, wobei gegebenenfalls 0,2 bis 5 vorzugsweise 0,2 bis 2,0 Gewichtsprozent, bezogen auf Wirksubstanz, der fakultativen Komponente (C) eingesetzt werden.

Die fakultativen, kosmetischen Hilfmittel als Komponente (C) sind handelsübliche Mittel, wie sie in der Haarkosmetik eingesetzt werden. In Betracht kommen z.B. Emulgatoren, die von den als Komponente (B) eingesetzten Tensiden der angegebenen Art verschieden sind (z.B. Fettsäureester von Glycerin, Aethylenglykol oder Propylenglykol, Fettsäuremonoäthanolamide, Fettalkohole, Lanolinalkohol, Fettalkyl-mono-, -di- und-triester der Phosphorsäure gegebenenfalls im Gemisch mit $C_{12}$-$C_{18}$-Fettalkoholen oder äthoxylierten $C_{12}$-$C_{18}$-Fettalkoholen), Schaumentwickler (z.B. Fettsäure-diäthanolamide, $C_{14}$-$C_{18}$-Alkyl-dimethylaminoxyde, äthoxylierte Fettalkylammoniumverbindungen), Konditioniermittel (z.B. Alkyl-Ammoniumverbindungen mit 1 oder 2 Fettalkyl- und 2 oder 3 $C_1$-$C_4$-Alkylresten), Verdickungsmittel (z.B. Hydroxypropylmethyl-Cellulose, Polyäthylenglykolfettsäureester, Fettsäureäthanolamide, Natrium- und Ammoniumchlorid), Konservierungsmittel (z.B. Methyl- und Aethylester der p-Hydroxybenzoesäure, 5-Brom-5-nitro-1,3-dioxan, Formaldehyd), Hautschutzmittel (z.B. Eiweisshydrolysate und Allantoine), ferner auch Riechstoffe und Perlglanzmittel.

Zur Herstellung der erfindungsgemässen Zusammensetzung wird z.B. so verfahren, dass man zum amphoteren Tensid als Komponente ($B_3$) das quaternäre Ammoniumsalz als Komponente (A) gibt, wobei man gleichzeitig, sofern das amphotere Tensid überschüssige Ladungen, insbesondere eine positive und zwei negative Ladungen aufweist, das Tensid ($B_3$) mit dem Ammoniumsalz (A) unter Ionenaustausch mindestens teilweise bei 10 bis 40°C umsetzt, anschliessend das nicht-ionogene Tensid als Komponente ($B_1$) und dann das anionische Tensid als Komponente ($B_2$) hinzu gibt, wobei man gleichzeitig das Tensid ($B_2$) mit dem Ammoniumsalz (A) unter Ionenaustausch bei 10 bis 40°C mindestens teilweise umsetzt, und dann das Reaktionsgemisch mit kosmetischen Hilfsmitteln als fakultative Komponente (C) gegebenenfalls versetzt und schliesslich den pH-Wert der erhaltenen Zusammensetzung auf 4 bis 8 einstellt und die Zusammensetzung mit entionisiertem Wasser verdünnt.

Somit werden bei der Herstellung der Zusammensetzung die polymeren, quaternären Ammoniumsalze als Komponente (A) mit dem anionischen Tensid als Komponente ($B_2$) und gegebenenfalls mit dem amphoteren Tensid als Komponente ($B_3$), sofern das amphotere Tensid ($B_3$) überschüssige negative Ladungen, insbesondere eine positive Ladung und zwei negative Ladungen aufweist, bei bevorzugten Temperaturen von 10 bis 40°C, vorzugsweise 20 bis 40°C, während 10 bis 120, vorzugsweise 30 bis 100, insbesondere 60 bis 90 Minuten, mindestens teilweise umgesetzt, wobei in der Regel ein Ueberschuss an anionisches Tensid ($B_2$) der Formeln (30) bis (70) und gegebenenfalls an amphoteres Tensid ($B_3$) mit einer positiven Ladung und zwei negativen Ladungen der Formeln (97) bis (102), (105), (108) oder (109), bezogen auf die quaternären Strukturelemente der Formeln (1), (5), (6), (8) oder (12) der homo- oder copolymeren Ammoniumsalze eingesetzt wird und die Umsetzung unter Ionenaustausch des Anions $Y_1^{\ominus}$ oder $Cl^{\ominus}$ der genannten Strukturelemente mit dem Anion $X_1^{\ominus}$ des anionischen Tensids und gegebenenfalls mit dem Anion $X_2^{\ominus}$ des amphoteren Tensids mit überschüssigen, negativen Ladungen der angegebenen Art mindestens teilweise erfolgt.

Vorzugsweise werden im Herstellungsverfahren der erfindungsgemässen Zusammensetzungen die Komponente (A) als wässrige, verdünnte, etwa 1 bis etwa 5 gewichtsprozentige Lösung und die Komponenten ($B_1$), ($B_2$) und ($B_3$) als wässrige, verdünnte, etwa 20 bis etwa 60 gewichtsprozentige Lösungen eingesetzt.

Nach dem Vermischen bzw. der Umsetzung der Komponente (A) mit den Komponenten ($B_1$), ($B_2$) und ($B_3$) und nach dem allfälligen Vermischen der fakultativen Komponente (C) wird der pH-Wert der Zusammensetzung auf 5 bis 8, vorzugsweise 5 bis 7, insbesondere 5 bis 6 eingestellt. Zu diesem Zweck werden im allgemeinen Natriumhydroxyd oder vorzugsweise Zitronensäure in der Regel ebenfalls in Form ihrer verdünnten, etwa 10 gewichtsprozentigen Lösungen eingesetzt. Anstelle einer Natriumhydroxydlösung kann auch z.B. eine wässrige Boraxlösung und anstelle von einer Zitronensäurelösung auch z.B. eine wässrige Milchsäurelösung eingesetzt werden.

Mit anionisiertem Wasser wird die Zusammensetzung ganz am Schluss auf 100 Gewichtsprozent gestellt.

Bei ihrer Verwendung in der Kosmetikindustrie werden die erfindungsgemässen Zusammensetzungen aus den Komponenten (A), ($B_1$), ($B_2$), ($B_3$) und gegebenenfalls (C) als haarkosmetische Mittel, insbesondere Haarshampoos eingesetzt.

Bei der Applikation der erfindungsgemässen Zusammensetzungen als Haarkosmetische Mittel, vorzugsweise auf keratinhaltigen Materialien, insbesondere menschlichem Haar, wird im Haarbehandlungsverfahren das haarkosmetische Mittel in Form eines Shampoos auf mit Leitungswasser angefeuchtetes Haar, in der Regel bei Raumtemperatur bis leicht erhöhter Temperatur, z.B. 20 bis 40°C, aufgebracht und das Haar anschliessend shampooniert und gleichzeitig konditioniert. Das so zu behandelnde Haar kann ebenfalls in Form von Perücken oder Toupets vorliegen.

Der wesentliche Vorteil der vorliegenden Erfindung liegt darin, dass die erfindungsgemässen Zusammensetzungen als haarkosmetische Mittel, welche neben polymeren, quaternären Ammoniumsalzen als Komponente (A) das ternäre Tensidsystem (B) aus den Komponenten ($B_1$), ($B_2$) und ($B_3$) in neuartiger Kombination enthalten, vollständig klare Formulierungen darstellen, worin die Komponenten (A), ($B_1$), ($B_2$) und ($B_3$) als komplexes System vorliegen, das durch Zusatz von Wasser präzipitiert, und dass bei der Applikation als haarkosmetische Mittel nicht nur eine gute Reinigung, sondern auch gleichzeitig infolge der besonders gleichmässigen Verteilung des präzipitierten komplexen Systems aus den Komponenten (A), ($B_1$), ($B_2$) und ($B_3$) bei der Verschäumung der erfindungsgemässen Zusammensetzung auf dem Haar ausgezeichnete Konditioniereffekte des behandelten Haars erzielt werden. So weisen die mit der erfindungsgemässen Zusammensetzung behandelten Haare in nassem Zustand eine sehr gute Entwirrbarkeit und in trockenem Zustand eine sehr gute Frisierwilligkeit und ansprechende antistatische Eigenschaften auf. Zudem werden sowohl in nassem als auch in trockenem

Zustand eine ausgezeichnete Kämmbarkeit und einen guten Griff erzielt. Bei wiederholter Verwendung der erfindungsgemässen Zusammensetzung ist ferner keinerlei unerwünschter Akkumuliereffekt unter Beeinträchtigung des Griffs, der Fülle und des Glanzes der behandelten Haare zu beobachten. Im Vergleich zu den Konditioniereffekten auf Haaren, die mit herkömmlichen kosmetischen Mitteln behandelt werden, weisen die mit den erfindungsgemässen haarkosmetischen Mitteln behandelten Haare somit in unerwarteter Weise auch bei äusserst niedriger Dosierung besonders gute Konditioniereffekte auf. Die üblichen kosmetischen Mittel enthalten zwar auch Tenside und quaternäre Ammoniumsalze, jedoch nicht in neuartiger und erfinderischer Kombination das ternäre Tensid System (B) aus ($B_1$) nicht-ionogener, ($B_2$) anionischer und ($B_3$) amphoterer Tenside gemäss vorliegender Erfindung. Als weiterer Vorteil sei erwähnt, dass beim Shampoonieren der Haare die erfindungsgemässen Zusammensetzungen einen Schaum der erwünschten, cremigen Beschaffenheit entwickeln, der stabiler, d.h. länger bestehen bleibt als beim Shampoonieren der Haare mit bekannten Zusammensetzungen. Durch die Schaumbeschaffenheit, auch Schaumstruktur genannt, ist auch eine gegenüber bekannten Zusammensetzungen verbesserte Reinigungswirkung zu erzielen. Als zusätzlicher Vorteil sei noch erwähnt, dass die Haarreinigung einstufig ohne Mitverwendung eines Nachspülmittels in einer aufwendigen zweiten Stufe erfolgt. Schliesslich sei darauf hingewiesen, dass die erfindungsgemässen Zusammensetzungen eine besonders hohe Hautverträglichkeit aufweist, die sich in einem angenehmen Hautgefühl nach erfolgter Applikation als Haarshampoo niederschlägt.

In der nachfolgenden Herstellungsvorschrift und in den nachfolgenden Beispielen angegebene Teile und Prozente sind Gewichtseinheiten.

<u>Herstellungsvorschrift für die Komponente (A)</u>

100 Teile eines im Handel erhältlichen Copolymerisates, das zu 75 Mol-% aus Strukturelementen der Formel (7) und zu 25 Mol-% aus Elementen der Formel

(110) 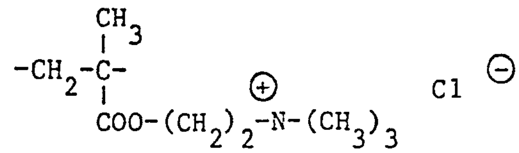

besteht, wobei das Molekulargewicht von 20 % des Copolymerisates zwischen $10^7$ und $10^9$ liegt, werden pulverisiert und mit einem Gemisch von 400 Teilen Methanol und 100 Teilen Wasser bei Raumtemperatur 30 Minuten lang gerührt. Weitere 500 Teile Methanol werden zugesetzt und das Gemisch während noch 5 Minuten unter Rühren gehalten und dann während einer Stunde stehen gelassen. Der gelige Brei wird dann in einer Drucknutsche unter einem Druck von 3 bar filtriert. Die gelige Masse wird anschliessend mit 100 Teilen Methanol aufgeschlämmt und wieder unter Druck abfiltriert. Nach dreimaliger Behandlung mit Methanol wird das Produkt während 24 Stunden unter vermindertem Druck bei 40°C getrocknet. Das Molekulargewicht von 45 % des erhaltenen, umgefällten Copolymerisats liegt zwischen $10^7$ und $10^9$.

Die molekulare Gewichtsverteilung wird mittels Gelpermeationschromatographie bestimmt, wobei als Trägermaterial FRACTOGEL® Typ OR-PVA eingesetzt wird, welches zweckmässig in Formamid als Lösungsmittel gequollen wird. Einzelheiten über das Trägermaterial sind aus dem Lehrbuch "Modern Size-Exclusion Chromatography" von W.W. Yau, J.J. Kirkland and D.D. Bly, Seiten 166 bis 173, Verlag John Wiley & Sons (1979) zu entnehmen. Als Detektor dient ein Differenzialrefraktometer.

Die Auswertung der GPC-Chromatogramme erfolgt anhand einer Eichkurve, die mit Polystyrol, Polymethylmethacrylat und den Standard-Polymeren SEPHADEX DEXTRAN® erstellt wird. Die Konzentrationen werden über die unkorrigierten Flächenprozente ermittelt, wobei die Summe aller eluierten Komponenten 100 % beträgt.

Beispiel 1:

Lösung A: 2 Teile des quaternären, polymeren Ammoniumsalzes gemäss Herstellungsvorschrift als Komponente (A) werden bei 20°C in kleinen Portionen innerhalb von 30 Minuten in 100 Teile entionisiertes Wasser eingetragen. Es entsteht eine viskose Lösung.

Lösung B: Zu einer Lösung von 50 Teilen des amphoteren Tensids der Formel (84) als Komponente ($B_3$) in 50 Teilen entionisiertem Wasser wird die Lösung A bei 20°C unter Rühren innerhalb von 30 Minuten gegeben.

Lösung C: Zur Lösung B wird eine Lösung von 80 Teilen des nicht-ionogenen Tensids der Formel (24) als Komponente ($B_1$) in 320 Teilen entionisiertem Wasser bei 20°C unter Rühren innerhalb von 10 Minuten gegeben.

Lösung D: Zur Lösung D wird eine Lösung von 10 Teilen des anionischen Tensids der Formel (41) als Komponente ($B_2$) in 10 Teilen entionisiertem Wasser bei 35°C unter Rühren innerhalb von etwa 90 Minuten gegeben, wobei gleichzeitig die Umsetzung der quaternären Strukturelemente der Formel (110) des polymeren Ammoniumsalzes mit dem eingesetzten, anionischen Tensid unter Ionenaustausch mindestens teilweise erfolgt. Hierbei ist darauf zu achten, dass allfällige Präzipitationen bei der Tensidzugabe gleich verschwinden. Nötigenfalls ist die Zugabezeit von 90 Minuten des anionischen Tensids entsprechend zu verlängern.

Erfindungsgemässe Zusammensetzungen: Die Lösung D wird mit 328 Teilen entionisiertem Wasser auf 950 Teile verdünnt, durch Zugabe einer 10 %igen, wässrigen Zitronensäurelösung auf den pH-Wert von 5,5 eingestellt und mit ca. 50 Teilen entionisiertem Wasser auf 1000 Teile verdünnt. Man erhält eine vollständig klare Zusammensetzung. Im

Reagenzglas fällt eine Probe der klaren Zusammensetzung durch Verdünnung mit Wasser im Gewichtsverhältnis 1:3 als sogenanntes Präzipitiersystem aus, wobei eine opale bis cremige Ausfällung erhalten wird.

Applikation der Zusammensetzung: Die erfindungsgemässe klare Zusammensetzung wird auf eine mit Leitungswasser angefeuchtete Perücke aus ungebleichtem und ungefärbtem, braunem Menschenhaar europäischer Herkunft bei 40°C in zweimaliger Applikation à 2 Shampoonierungen im sogenannten Halbseitentest aufgebracht, worauf das Perückenhaar bei dieser Temperatur shampooniert und gleichzeitig konditioniert wird. Nach jeder Shampoonierung wird das Haar mit Leitungswasser von 20 bis 40°C kurz nachgespült. Im Halbseitentest wird nur die eine Hälfte der Perücke mit der vorstehend genannten Lösung shampooniert und konditioniert, während die andere Hälfte der Perücke mit einer Lösung unter gleichen Bedingungen shampooniert wird, die kein erfindungsgemässes Gemisch aus dem Ammoniumsalz (A) und dem ternären Tensidsystem (B), sondern nur das ternäre Tensidsystem (B) enthält. In dieser sogenannten Leerformulierung wird die Menge an Ammoniumsalz durch eine entsprechende Menge an nicht-ionogenem Tensid ersetzt, so dass im vorliegenden Fall die Leerformulierung als Vergleich 8,2% des nicht-ionogenen Tensids der Formel (24) 1% des anionischen Tensids der Formel (41) und 5% des amphoteren Tensids der Formel (84) enthält, wobei die Leerformulierung ebenfalls mit der wässrigen 10%igen Zitronensäurelösung auf den pH-Wert von 5,5 eingestellt wird. Beim Schampoonieren der Haare kann eine cremige Beschaffenheit des Schaumes beobachtet werden, die beim Schampoonieren mit der Leerformulierung nicht vorhanden ist. Nach jeder Applikation wird die Nass- und Trockenkämmbarkeit der erfindungsgemäss behandelten Perückenhälfte im Vergleich zu der mit der Leerformulierung behandelten Perückenhälfte mit Hilfe der nachfolgenden Notenskala beurteilt.

+3 viel besser als die Leerformulierung
+2 besser als die Leerformulierung
+1 etwas besser als die Leerformulierung
0 kein Unterschied zur Leerformulierung

-1 etwas schlechter als die Leerformulierung

-2 schlechter als die Leerformulierung

-3 viel schlechter als die Leerformulierung.

Bei dieser Notenskala kann eine halbe Einheit noch wahrgenommen werden.

Die erzielten Kämmbarkeitsnoten sind in der nachfolgenden Tabelle I zusammengefasst.

Tabelle I

| | nach der 1. Applikation | nach der 2. Applikation |
|---|---|---|
| Nasskämmbarkeit | +(1-2) | +(1-2) |
| Trockenkämmbarkeit | +(0-1) | +(0-1) |

Beispiel 2: Man verfährt wie in Beispiel 1 angegeben, setzt jedoch 65 Teile des nicht-ionogenen Tensids der Formel (24) als Komponente ($B_1$) in 260 Teilen entionisiertem Wasser, 40 Teile des anionischen Tensids der Formel (41) als Komponente ($B_2$) in 40 Teilen entionisiertem Wasser und 65 Teile des amphoteren Tensids der Formel (84) als Komponente ($B_3$) in 65 Teilen entionisiertem Wasser ein und verdünnt die Lösung D auf insgesamt 1000 Teile mit ca. 363 Teilen entionisiertem Wasser. Wie im Beispiel 1 angegeben ist die erhaltene Zusammensetzung vollständig klar und fällt durch Verdünnung mit Wasser aus.

Bei der Applikation der Zusammensetzung auf Haar wie in Beispiel 1 angegeben erzielt man die in der nachfolgenden Tabelle II angegebenen Kämmbarkeitsnoten.

Tabelle II

| | nach der 1. Applikation | nach der 2. Applikation |
|---|---|---|
| Nasskämmbarkeit | +(1-2) | +2 |
| Trockenkämmbarkeit | +(0-1) | +(0-1) |

Beispiel 3: Man verfährt wie in Beispiel 1 angegeben, setzt jedoch 40 Teile des nicht-ionogenen Tensids der Formel (24) als Komponente ($B_1$) in 160 Teilen entionisiertem Wasser, 57 Teile des anionischen Tensids der Formel (41) als Komponente ($B_2$) in 57 Teilen entionisiertem Wasser und 90 Teile des amphoteren Tensids der Formel (84) als Komponente ($B_3$) in 90 Teilen entionisiertem Wasser ein und verdünnt die Lösung D auf 1000 Teile mit ca. 404 Teilen entionisiertem Wasser. Wie im Beispiel 1 angegeben ist die erhaltene Zusammensetzung vollständig klar und fällt durch Verdünnung mit Wasser aus.

Bei der Applikation der Zusammensetzung auf Haar wie in Beispiel 1 angegeben, erzielt man die in der nachfolgenden Tabelle III angegebenen Kämmbarkeitsnoten.

Tabelle III

| | nach der 1. Applikation | nach der 2. Applikation |
|---|---|---|
| Nasskämmbarkeit | +2 | +(2-3) |
| Trockenkämmbarkeit | +1 | +1 |

Beispiel 4: Man verfährt wie in Beispiel 1 angegeben, setzt jedoch 100 Teile des nicht-ionogenen Tensids der Formel (24) als Komponente ($B_1$) in 400 Teilen entionisiertem Wasser, 4 Teile des anionischen Tensids der Formel (41) als Komponente ($B_2$) in 4 Teilen entionisiertem Wasser und 30 Teile des amphoteren Tensids der Formel (84) als Komponente ($B_3$) in 30 Teilen entionisiertem Wasser ein und verdünnt die Lösung D auf 1000 Teile mit ca. 330 Teilen entionisiertem Wasser. Wie in Beispiel 1 angegeben ist die erhaltene Zusammensetzung vollständig klar und fällt durch Verdünnung mit Wasser aus.

Bei der Applikation der Zusammensetzung auf Haar erzielt man ähnliche Ergebnisse, wie sie in Beispiel 1 angegeben sind.

Beispiel 5: Man verfährt wie in Beispiel 1 angegeben, setzt jedoch 30 Teile des nicht-ionogenen Tensids der Formel (24) als Komponente ($B_1$) in 120 Teilen entionisiertem Wasser, 45 Teile des anionischen Tensids der Formel (41) als Komponente ($B_2$) in 45 Teilen entionisiertem Wasser und 100 Teile des amphoteren Tensids der Formel (84) als Komponente ($B_3$) in 100 Teilen entionisiertem Wasser ein und verdünnt die Lösung D auf 1000 Teile mit ca. 458 Teilen entionisiertem Wasser. Wie in Beispiel 1 angegeben ist die erhaltene Zusammensetzung vollständig klar und fällt durch Verdünnung mit Wasser aus.

Bei der Applikation der Zusammensetzung auf Haar erzielt man ähnliche Ergebnisse, wie sie in Beispiel 3 angegeben sind.

Beispiel 6: Man verfährt wie in Beispiel 1 angegeben, setzt jedoch 70 Teile des nicht-ionogenen Tensids der Formel (20) als Komponente ($B_1$) in 280 Teilen entionisiertem Wasser, 40 Teile des anionischen Tensids der Formel (32) als Komponente ($B_2$) in 40 Teilen entionisiertem Wasser und 40 Teile des amphoteren Tensids der Formel (84) als Komponente ($B_3$) in 40 Teilen entionisiertem Wasser ein und verdünnt die Lösung D auf 1000 Teile mit ca. 388 Teilen entionisiertem Wasser. Wie in Beispiel 1 angegeben ist die erhaltene Zusammensetzung vollständig klar und fällt durch Verdünnung mit Wasser aus.

Bei der Applikation der Zusammensetzung auf Haar wie in Beispiel 1 angegeben, erzielt man die in der nachfolgenden Tabelle IV angegebenen Kämmbarkeitsnoten.

Tabelle IV

| | nach der 1. Applikation | nach der 2. Applikation |
|---|---|---|
| Nasskämmbarkeit | +(1-2) | +2 |
| Trockenkämmbarkeit | +1 | +1 |

Beispiel 7: Man verfährt wie in Beispiel 1 angegeben, setzt jedoch 80 Teile des nicht-ionogenen Tensids der Formel (20) als Komponente ($B_1$) in 320 Teilen entionisiertem Wasser, 30 Teile des anionischen Tensids der Formel (41) als Komponente ($B_2$) in 30 Teilen entionisiertem Wasser und 40 Teilen des amphoteren Tensids der Formel (84) als Komponente ($B_3$) in 40 Teilen entionisiertem Wasser ein und verdünnt die Lösung D auf 1000 Teile mit ca. 358 Teilen entionisiertem Wasser. Wie in Beispiel 1 angegeben ist die erhaltene Zusammensetzung vollständig klar und fällt durch Verdünnung mit Wasser aus.

Bei der Applikation der Zusammensetzung auf Haar erzielt man ähnliche Ergebnisse, wie sie in Beispiel 6 angegeben sind. Das gleiche gilt auch, wenn man zur Lösung D 20 Teile Cetylalkohol als Emulgator (fakultative Komponente (C)) gibt und die Lösung D auf 1000 Teile mit ca. 338 Teilen entionisiertem Wasser verdünnt.

Beispiel 8: Man verfährt wie in Beispiel 1 angegeben, setzt jedoch 40 Teile des nicht-ionogenen Tensids der Formel (19) als Komponente ($B_1$) in 160 Teilen entionisiertem Wasser, 10,5 Teile des anionischen Tensids der Formel (41) als Komponente ($B_2$) in 10,5 Teilen entionisiertem Wasser und 90 Teile des amphoteren Tensides der Formel (84) als

Komponente ($B_3$) in 90 Teilen entionisiertem Wasser ein, gibt zur Lösung D 5 Teile Tetradecyltrimethyl-Ammoniumchlorid als Konditioniermittel (fakultative Komponente (C)) und verdünnt die Lösung D auf 1000 Teile mit ca. 492 Teilen entionisiertem Wasser. Wie in Beispiel 1 angegeben ist die erhaltene Zusammensetzung vollständig klar und fällt durch Verdünnung mit Wasser aus.

Bei der Applikation der Zusammensetzung auf Haar erzielt man ähnliche Ergebnisse, wie sie in Beispiel 6 angegeben sind.

Beispiel 9: Man verfährt wie in Beispiel 1 angegeben, setzt jedoch 100 Teile des nicht-ionogenen Tensides der Formel (19) als Komponente ($B_1$) in 400 Teilen entionisiertem Wasser, 57 Teile des anionischen Tensids der Formel (41) als Komponente ($B_2$) in 57 Teilen entionisiertem Wasser und 90 Teile des amphoteren Tensids der Formel(84) als Komponente ($B_3$) in 90 Teilen entionisiertem Wasser ein, gibt zur Lösung D 5 Teile Tetradecyltrimethyl-Ammoniumchlorid als Konditioniermittel, (fakultative Komponente (C)) und verdünnt die Lösung D auf 1000 Teile mit ca. 99 Teilen entionisiertem Wasser. Wie in Beispiel 1 angegeben ist die erhaltene Zusammensetzung vollständig klar und fällt durch Verdünnung mit Wasser aus.

Bei der Applikation der Zusammensetzung auf Haar wie in Beispiel 1 angegeben erhält man die in der nachfolgenden Tabelle V angegebenen Kämmbarkeitsnoten.

Tabelle V:

| | nach der 1. Applikation | nach der 2. Applikation |
|---|---|---|
| Nasskämmbarkeit | +(2-3) | +(2-3) |
| Trockenkämmbarkeit | +(1-2) | +(1-2) |

Beispiel 10: Man verfährt wie in Beispiel 1 angegeben, setzt jedoch 5 Teile eines polymerisierten Dimethyldiallylammoniumchlorids eines Molekulargewichtes von 40 000 bis 60 000 als Komponente (A), das wiederkehrende Strukturelemente der Formel (6) aufweist, worin n zum Teil 1 und zum Teil 2 bedeutet in 100 Teilen entionisiertem Wasser, 100 Teile des nicht-ionogenen Tensides der Formel (24) als Komponente ($B_1$) in 400 Teilen entionisiertem Wasser, 22 Teile des anionischen Tensides der Formel (41) als Komponente ($B_2$) in 22 Teilen entionisiertem Wasser und 35 Teile des amphoteren Tensides der Formel (84) als Komponente ($B_3$) in 35 Teilen entionisiertem Wasser ein und verdünnt die Lösung D auf 1000 Teile mit ca. 281 Teilen entionisiertem Wasser. Wie in Beispiel 1 angegeben ist die erhaltene Zusammensetzung vollständig klar und fällt durch Verdünnung mit Wasser aus.

Bei der Applikation der Zusammensetzung auf Haar wie in Beispiel 1 angegeben erzielt man die in der nachfolgenden Tabelle VI angegebenen Kämmbarkeitsnoten.

Tabelle VI :

| | nach der 1. Applikation | nach der 2. Applikation |
|---|---|---|
| Nasskämmbarkeit | +(2-3) | +(2-3) |
| Trockenkämmbarkeit | +(1-2) | +(1-2) |

Beispiel 11: Man verfährt wie in Beispiel 1 angegeben, setzt jedoch 5 Teile eines polymerisierten Dimethyldiallylammoniumchlorids eines Molekulargewichtes von 40 000 bis 60 000 als Komponente (A), das wiederkehrende Strukturelemente der Formel (6) aufweist, worin n zum

Teil 1 und zum Teil 2 bedeutet, in 100 Teilen entionisiertem Wasser, 73 Teile des nicht-ionogenen Tensides der Formel (20) als Komponente ($B_1$) in 292 Teilen entionisiertem Wasser, 44 Teile des anionischen Tensides der Formel (41) als Komponente ($B_2$) in 44 Teilen entionisiertem Wasser und 30 Teilen des amphoteren Tensids der Formel (84) als Komponente ($B_3$) in 30 Teilen entionisiertem Wasser ein, gibt zur Lösung D 20 Teile eines Polyäthylenglykoldiester von Stearinsäure mit einem durchschnittlichen Aethoxylierungsgrad von 140 bis 160 als Verdickungsmittel (fakultative Komponente (C)) und verdünnt die Lösung D auf 1000 Teile mit ca. 362 Teilen entionisiertem Wasser. Wie im Beispiel 1 angegeben ist die erhaltene Zusammensetzung vollständig klar und fällt durch Verdünnung mit Wasser aus.

Bei der Applikation der Zusammensetzung auf Haar erzielt man ähnliche Ergebnisse wie sie in Beispiel 10 angegeben sind.

Beispiel 12: Man verfährt wie in Beispiel 1 angegeben, setzt jedoch 5 Teile eines polymerisierten Dimethyldiallylammoniumchlorids eines Molekulargewichtes von 40 000 bis 60 000 als Komponente (A), das wiederkehrende Strukturelemente der Formel (6) aufweist, worin n zum Teil 1 und zum Teil 2 bedeutet, in 100 Teilen entionisiertem Wasser, 100 Teile des nicht-ionogenen Tensids der Formel (24) als Komponente ($B_1$) in 400 Teilen entionisiertem Wasser, 103 Teile des anionischen Tensides der Formel (41) als Komponente ($B_2$) in 85 Teilen entionisiertem Wasser und 53 Teile des amphoteren Tensides der Formel (84) als Komponente ($B_3$) in 85 Teilen entionisiertem Wasser ein und verdünnt die Lösung D auf 1000 Teile mit ca. 69 Teilen entionisiertem Wasser. Wie in Beispiel 1 angegeben ist die erhaltene Zusammensetzung vollständig klar und fällt durch Verdünnung mit Wasser aus.

Bei der Applikation der Zusammensetzung auf Haar erzielt man ähnliche Ergebnisse, wie sie in Beispiel 10 angegeben sind.

Beispiel 13: Man verfährt wie in Beispiel 1 angegeben, setzt jedoch 5 Teile einer Poly(dimethylbutenylammoniumchlorid)-α,γ-bis-(triethanolammoniumchlorid)-Verbindung eines Molekulargewichtes von etwa 2000, die der Formel (5) entspricht, worin $m_4$ 12 bedeutet, als Komponente (A) in 100 Teilen entionisiertem Wasser 40 Teile des nicht-ionogenen Tensids der Formel (24) als Komponente ($B_1$) in 160 Teilen entionisiertem Wasser, 60 Teile des anionischen Tensids der Formel (41) als Komponente ($B_2$) in 60 Teilen entionisiertem Wasser und 90 Teile des amphoteren Tensides der Formel (84) als Komponente ($B_3$) in 90 Teilen entionisiertem Wasser ein und verdünnt die Lösung D auf 1000 Teile mit ca. 395 Teilen entionisiertem Wasser. Wie in Beispiel 1 angegeben ist die erhaltene Zusammensetzung vollständig klar und fällt durch Verdünnung mit Wasser aus.

Bei der Applikation der Zusammensetzung auf Haar erzielt man ähnliche Ergebnisse, wie sie in Beispiel 10 angegeben sind.

Beispiel 14: Man verfährt wie in Beispiel 1 angegeben, setzt jedoch 40 Teile des nicht-ionogenen Tensids der Formel (24) als Komponente ($B_1$) in 160 Teilen entionisiertem Wasser, 50 Teile des anionischen Tensides der Formel (32) als Komponente ($B_2$) in 50 Teilen entionisiertem Wasser und 90 Teile des amphoteren Tensids der Formel (84) als Komponente ($B_3$) in 90 Teilen entionisiertem Wasser ein, gibt zur Lösung D 5 Teile Dioctadecyldimethylammoniumchlorid als Konditioniermittel (fakultative Komponente (C)) und verdünnt die Lösung D auf 1000 Teile mit ca. 413 Teilen entionisiertem Wasser. Wie in Beispiel 1 angegeben ist die erhaltene Zusammensetzung vollständig klar und fällt durch Verdünnung mit Wasser aus.

Bei der Applikation der Zusammensetzung auf Haar wie in Beispiel 1 angegeben, erzielt man die in der nachfolgenden Tabelle VII angegebenen Kämmbarkeitsnoten.

Tabelle VII:

| | nach der 1. Applikation | nach der 2. Applikation |
|---|---|---|
| Nasskämmbarkeit | +(2-3) | +(2-3) |
| Trockenkämmbarkeit | +(1-2) | +2 |

Beispiel 15: Man verfährt wie in Beispiel 1 angegeben, setzt jedoch 40 Teile des nicht-ionogenen Tensids der Formel (20) als Komponente ($B_1$) in 160 Teilen entionisiertem Wasser, 50 Teile des anionischen Tensids der Formel (41) als Komponente ($B_2$) in 50 Teilen entionisiertem Wasser und 40 Teile des amphoteren Tensids der Formel (84) als Komponente ($B_3$) in 40 Teilen entionisiertem Wasser ein, gibt zur Lösung D 5 Teile Oelsäure als fakultative Komponente (C) und verdünnt die Lösung D auf 1000 Teile mit ca. 513 Teilen entionisiertem Wasser. Wie in Beispiel 1 angegeben ist die erhaltene Zusammensetzung vollständig klar und fällt durch Verbindung mit Wasser aus.

Bei der Applikation der Zusammensetzung auf Haar wie in Beispiel 1 angegeben, erzielt man die in der nachfolgenden Tabelle VIII angegebenen Kämmbarkeitsnoten.

Tabelle VIII:

| | nach der 1. Applikation | nach der 2. Applikation |
|---|---|---|
| Nasskämmbarkeit | +2 | +3 |
| Trockenkämmbarkeit | +1 | +(1-2) |

Beispiel 16: Man verfährt wie in Beispiel 1 angegeben, setzt jedoch 40 Teile des nicht-ionogenen Tensids der Formel (22) als Komponente ($B_1$) in 160 Teilen entionisiertem Wasser, 35 Teile des anionischen Tensids der Formel (41) als Komponente ($B_2$) in 35 Teilen entionisiertem Wasser und 90 Teile des amphoteren Tensids der Formel (84) als Komponente ($B_3$) in 90 Teilen entionisiertem Wasser ein und verdünnt die Lösung D auf 1000 Teile mit ca. 448 Teilen entionisiertem Wasser. Wie in Beispiel 1 angegeben ist die erhaltene Zusammensetzung vollständig klar und fällt durch Verdünnung mit Wasser aus.

Bei der Applikation der Zusammensetzung auf Haar wie in Beispiel 1 angegeben erzielt man die in der nachfolgenden Tabelle IX angegebenen Kämmbarkeitsnoten.

Tabelle IX

| | nach der 1. Applikation | nach der 2. Applikation |
|---|---|---|
| Nasskämmbarkeit | +2 | +(2-3) |
| Trockenkämmbarkeit | +1 | +(1-2) |

Beispiel 17: Man verfährt wie in Beispiel 1 angegeben, setzt jedoch 40 Teile des nicht-ionogenen Tensids der Formel (24) als Komponente ($B_1$) in 160 Teilen entionisiertem Wasser, 57 Teile des anionischen Tensids der Formel (41) als Komponente ($B_2$) in 57 Teilen entionisiertem Wasser und 90 Teile des amphoteren Tensids der Formel (84) als Komponente ($B_3$) in 90 Teilen entionisiertem Wasser ein, gibt zur Lösung D 5 Teile Tetradecyltrimethylammoniumchlorid als Konditioniermittel, 2 Teile einer 10%igen, wässrigen Lösung aus 5-Brom-5-nitro-1,3-dioxan als Konservierungsmittel und 0,1 Teile eines handelsüblichen Riechstoffes (fakultative Komponenten (C)) und verdünnt die

Lösung D auf 1000 Teile mit ca. 397 Teilen entionisiertem Wasser. Wie in Beispiel 1 angegeben ist die erhaltene Zusammensetzung vollständig klar und fällt durch Verdünnung mit Wasser aus.

Bei der Applikation der Zusammensetzung auf Haar erzielt man ähnliche Ergebnisse, wie sie in Beispiel 14 angegeben sind.

Beispiel 18: Man verfährt wie in Beispiel 1 angegeben, setzt jedoch 5 Teile eines Cellulose-Derivates eines Molekulargewichtes von ca. 400 000 als Komponente (A), das quaternäre Substituenten der Formeln (1) und gegebenenfalls (2), (3) und (4) aufweist, in 100 Teilen entionisiertem Wasser, 65 Teile des nicht-ionogenen Tensids der Formel (20) als Komponente ($B_1$) in 400 Teilen entionisiertem Wasser, 50 Teile des anionischen Tensides der Formel (40) als Komponente ($B_2$) in 50 Teilen entionisiertem Wasser und 65 Teile des amphoteren Tensides der Formel (84) als Komponente ($B_3$) in 65 Teilen entionisiertem Wasser ein, gibt zur Lösung D 2 Teile einer 10%igen, wässrigen Lösung aus 5-Brom-5-nitro-1,3-dioxan als Konservierungsmittel (fakultative Komponente (C)), und verdünnt die Lösung D auf 1000 Teile mit ca. 200 Teilen entionisiertem Wasser, wobei durch Zusatz der 10%igen, wässrigen Zitronensäurelösung der pH-Wert der Zusammensetzung jedoch auf 6,0 eingestellt wird. Wie in Beispiel 1 angegeben ist die erhaltene Zusammensetzung vollständig klar und fällt durch Verdünnung mit Wasser aus.

Bei der Applikation der Zusammensetzung auf Haar erzielt man nach der 1. Applikation die Note +1 für die Nasskämmbarkeit. Zudem wird mit Hilfe der gleichen Notenskala, wie sie für die Nass- und Trockenkämmbarkeit im Beispiel 1 angewendet wird, der Griff (in nassem Zustand) und die Entwirrbarkeit (ebenfalls in nassem Zustand) beurteilt. Man erhält nach der 1. Applikation die Note +(2-3) für den Griff und die Note +1 für die Entwirrbarkeit.

Beispiel 19: Man verfährt wie in Beispiel 1 angegeben, setzt jedoch 65 Teile des nicht-ionogenen Tensids der Formel (20) als Komponente ($B_1$) in 160 Teilen entionisiertem Wasser, 35 Teile des anionischen Tensids der Formel (50) als Komponente ($B_2$) in 50 Teilen entionisiertem Wasser und 65 Teile des amphoteren Tensids der Formel (84) als Komponente ($B_3$) in 65 Teilen entionisiertem Wasser ein, gibt zur Lösung D 2 Teile einer 10%igen, wässrigen Lösung aus 5-Brom-5-nitro-1,3-dioxan als Konservierungsmittel (fakultative Komponente (C)) und verdünnt die Lösung D auf 1000 Teile mit ca. 456 Teilen entionisiertem Wasser. Wie in Beispiel 1 angegeben ist die erhaltene Zusammensetzung vollständig klar und fällt durch Verdünnung mit Wasser aus.

Bei der Applikation der Zusammensetzung erzielt man nach der 1. Applikation auf dem Haar in nassem Zustand die Noten +3 für die Kämmbarkeit, +(2-3) für den Griff und +3 für die Entwirrbarkeit.

Beispiel 20: Man verfährt wie in Beispiel 1 angegeben, setzt jedoch 65 Teile des nicht-ionogenen Tensids der Formel (20) als Komponente ($B_1$) in 160 Teilen entionisiertem Wasser, 30 Teile des anionischen Tensids der Formel (40) als Komponente ($B_2$) in 30 Teilen entionisiertem Wasser und 65 Teile des amphoteren Tensids der Formel (98) als Komponente ($B_3$) in 90 Teilen entionisiertem Wasser ein, gibt zur Lösung D 2 Teile einer 10%igen, wässrigen Lösung aus 5-Brom-5-nitro-1,3-dioxan als Konservierungsmittel (fakultative Komponente (C)) und verdünnt die Lösung D auf 1000 Teile mit ca. 456 Teilen entionisiertem Wasser. Wie in Beispiel 1 angegeben ist die erhaltene Zusammensetzung vollständig klar und fällt durch Verdünnung mit Wasser aus.

Bei der Applikation der Zusammensetzung erzielt man nach der 1. Applikation auf dem Haar in nassem Zustand die Noten +3 für die Kämmbarkeit, +(2-3) für den Griff und +3 für die Entwirrbarkeit.

Beispiel 21: Man verfährt wie in Beispiel 1 angegeben, setzt jedoch 65 Teile des nicht-ionogenen Tensids der Formel (20) als Komponente ($B_1$) in 160 Teilen entionisiertem Wasser, 35 Teile des anionischen Tensids der Formel (40) als Komponente ($B_2$) in 35 Teilen entionisiertem Wasser und 65 Teile des amphoteren Tensids der Formel (101) als Komponente ($B_3$) in 90 Teilen entionisiertem Wasser ein, gibt zur Lösung D 2 Teile einer 10%igen, wässrigen Lösung aus 5-Brom-5-nitro-1,3-dioxan als Konservierungsmittel (fakultative Komponente (C)) und verdünnt die Lösung D auf 1000 Teile mit ca. 446 Teilen entionisiertem Wasser. Wie in Beispiel 1 angegeben ist die Zusammensetzung vollständig klar und fällt durch Verdünnung mit Wasser aus.

Bei der Applikation der Zusammensetzung erzielt man nach der 1. Applikation auf dem Haar in nassem Zustand die Noten +(2-3) für die Kämmbarkeit, +(1-2) für den Griff und +(2-3) für die Entwirrbarkeit.

Beispiel 22: Man verfährt wie in Beispiel 1 angegeben, setzt jedoch 1 Teil (statt 2 Teile) des quaternären, polymeren Ammoniumsalzes gemäss Herstellungsvorschrift als Komponente (A) 40 Teile eines Gemisches der nicht-ionogenen Tenside der Formeln (27) und (28) als Komponente ($B_1$) in 160 Teilen entionisiertem Wasser, 70 Teile des anionischen Tensids der Formel (58), worin p und n für 2 und $T_1$ für den Rest der Ricinolsäure stehen, als Komponente ($B_2$) in 46 Teilen entionisiertem Wasser und 30 Teilen des amphoteren Tensids der Formel (84) als Komponente ($B_3$) in 70 Teilen entionisiertem Wasser ein, gibt zur Lösung D 20 Teile Natriumchlorid als Verdickungsmittel, 2 Teile einer 10%igen Lösung von 5-Brom-5-nitro-1,3-dioxan als Konservierungsmittel, 10 Teile einer 0,1%igen, wässrigen Lösung aus C.I. Acid Green 1 (C.I. Nr. 10020) als Farbstoff und 0,1 Teile eines handelsüblichen Riechstoffes (fakultative Komponenten (C)) und verdünnt die Lösung D auf 1000 Teile mit ca. 451 Teilen entionisiertem Wasser. Wie in Beispiel 1 angegeben ist die erhaltene Zusammensetzung vollständig klar und fällt durch Verdünnung mit Wasser aus.

Bei der Applikation der Zusammensetzung erzielt man nach der 1. Applikation auf dem Haar in nassem Zustand die Noten +(2-3) für die Kämmbarkeit, +(1-2) für den Griff und +2 für die Entwirrbarkeit.

Beispiel 23: Man verfährt wie in Beispiel 1 angegeben, setzt jedoch 5 Teile eines im Handel erhältlichen, aus der Guarpflanze gewonnenes Polygalactomannan als Komponente (A), das quaternäre Reste der Formeln (1) und gegebenenfalls (2), (3) und (4) aufweist (JAGUAR C 13 S®), in 100 Teilen entionisiertem Wasser, 10 Teile des nicht-ionogenen Tensids der Formel (24) als Komponente ($B_1$) in 40 Teilen entionisiertem Wasser, 56 Teile des anionischen Tensids der Formel (40) als Komponente ($B_2$) in 144 Teilen entionisiertem Wasser und 12 Teile des amphoteren Tensids der Formel (84) als Komponente ($B_3$) in 28 Teilen entionisiertem Wasser ein und verdünnt die Lösung D auf 1000 Teile mit ca. 605 Teilen entionisiertem Wasser, wobei jedoch durch Zusatz einer 10%igen, wässrigen Natriumhydroxydlösung der pH-Wert der Zusammensetzung auf 7,0 eingestellt wird.

Bei der Applikation der Zusammensetzung erzielt man nach der 1. Applikation auf dem Haar in nassem Zustand die Noten +(2-3) für die Kämmbarkeit, +2 für den Griff und +(2-3) für die Entwirrbarkeit.

Beispiel 24: Man verfährt wie in Beispiel 1 angegeben, setzt jedoch 5 Teile JAGUAR C 13 S® in 100 Teilen entionisiertem Wasser, 10 Teile des nicht-ionogenen Tensids der Formel (24) als Komponente ($B_1$) in 40 Teilen entionisiertem Wasser, 50 Teile des anionischen Tensids der Formel (40) als Komponente ($B_2$) in 128 Teilen

entionisiertem Wasser und 38 Teile des amphoteren Tensids der Formel (84) als Komponente ($B_3$) in 88 Teilen entionisiertem Wasser ein und verdünnt die Lösung D auf 1000 Teile mit ca. 541 Teilen entionisiertem Wasser, wobei durch Zusatz einer 10%igen, wässrigen Zitronensäurelösung der pH-Wert der Zusammensetzung auf jedoch 6,4 eingestellt wird.

Bei der Applikation der Zusammensetzung erzielt man nach der 1. Applikation auf dem Haar in nassem Zustand die Noten +3 für die Kämmbarkeit, +(2-3) für den Griff und +(2-3) für die Entwirrbarkeit.

Beispiel 25: Man verfährt wie in Beispiel 1 angegeben, setzt jedoch 5 Teile JAGUAR C 13 S® in 100 Teilen entionisiertem Wasser, 20 Teile des nicht-ionogenen Tensids der Formel (24) als Komponente ($B_1$) in 80 Teilen entionisiertem Wasser, 40 Teile des anionischen Tensids der Formel (40) als Komponente ($B_2$) in 103 Teilen entionisiertem Wasser und 40 Teile des amphoteren Tensids der Formel (84) als Komponente ($B_3$) in 93 Teilen entionisiertem Wasser ein und verdünnt die Lösung D auf 1000 Teile mit ca. 519 Teilen entionisiertem Wasser, wobei jedoch ohne Zusatz von Zitronensäure oder Natriumhydroxyd der pH-Wert der Zusammensetzung 6,6 beträgt.

Bei der Applikation der Zusammensetzung erzielt man nach der 1. Applikation auf dem Haar in nassem Zustand die Noten +(2-3) für die Kämmbarkeit, +2 für den Griff und +2 für die Entwirrbarkeit.

Beispiele 26 bis 38: Aehnliche Ergebnisse wie in Beispiel 18 angegeben werden erzielt, wenn die in den nachfolgenden Tabellen X und XI angegebenen Zusammensetzungen eingesetzt werden.

Tabelle X:

| Beispiel Nr. | 26 | 27 | 28 | 29 | 30 | 31 |
|---|---|---|---|---|---|---|
| Komponente (A)<br>Teile (Wirksubstanz) des in Beispiel 1 angegebenen, quaternären, polymeren Ammoniumsalzes | 2 | 2 | 2 | 2 | 2 | 2 |
| Komponente ($B_1$)<br>Teile (Wirksubstanz) des nichtionogenen Tensides der Formel | | | | | | |
| (13), worin $T_1$ für $C_{11}$-Alkyl und p für 10 stehen | 25 | - | - | - | - | - |
| (14), worin $T_1$ für $C_{17}$-Alkyl und p für 12 stehen | - | 25 | - | - | - | - |
| (17), worin p für 12 und s für 4 stehen | - | - | 65 | - | - | - |
| (18) | - | - | - | - | 65 | - |
| (20) | 40 | 55 | - | 65 | - | 65 |
| Komponente ($B_2$)<br>Teile (Wirksubstanz) des anionischen Tensides der Formel | | | | | | |
| (31) | - | - | - | 25 | - | - |
| (37) | - | - | - | - | - | 25 |
| (40) | 25 | 25 | 25 | - | 35 | - |
| Komponente ($B_3$)<br>Teile (Wirksubstanz) des amphoteren Tensides der Formel | | | | | | |
| (84) | 65 | 65 | 65 | 65 | 65 | 65 |

Tabelle XI:

| Beispiel Nr. | 32 | 33 | 34 | 35 | 36 | 37 | 38 |
|---|---|---|---|---|---|---|---|
| Komponente (A)<br>Teile (Wirksubstanz) des in Beispiel 1 angegebenen, quaternären, polymeren Ammoniumsalzes | 2 | 2 | 2 | 2 | 2 | 2 | 2 |
| Komponente ($B_1$)<br>Teile (Wirksubstanz) des nicht-ionogenen Tensides der Formel | | | | | | | |
| (20) | 65 | 65 | 40 | 65 | 40 | 40 | 40 |
| Komponente ($B_2$)<br>Teile (Wirksubstanz) des anionischen Tensides der Formel | | | | | | | |
| (31) | - | - | 40 | - | 40 | - | - |
| (40) | - | - | - | 35 | - | 25 | 25 |
| (52) | 50 | - | - | - | - | - | - |
| (55) | - | 35 | - | - | - | - | - |
| Komponente ($B_3$)<br>Teile (Wirksubstanz) des amphoteren Tensides der Formel | | | | | | | |
| (81) | - | - | 80 | - | - | - | - |
| (83) | - | - | - | 65 | - | - | - |
| (84) | 65 | 65 | - | - | - | - | - |
| (92) | - | - | - | - | 45 | - | - |
| (94) | - | - | - | - | - | 60 | - |
| (102) | - | - | - | - | - | - | 60 |

Die in den Tabellen X und XI angegebenen Zusammensetzungen werden wie in Beispiel 1 hergestellt, wobei die Komponente (A) ebenfalls als 2%ige, wässrige Lösung, die nicht-ionogenen Tenside als Komponente ($B_1$) und die anionischen Tenside als Komponente ($B_2$) ebenfalls als 50%ige, wässrige Lösungen und die amphoteren Tenside als Komponente ($B_3$) ebenfalls als 70%ige, wässrige Lösungen eingesetzt werden und die Lösungen D mit entsprechenden Mengen entionisiertem Wasser auf insgesamt 1000 Teile verdünnt werden, wobei durch Zusatz der 10%igen, wässrigen Zitronensäurelösung der pH-Wert der Zusammensetzungen ebenfalls auf 5,5 eingestellt wird. Alle Zusammensetzungen der Tabellen X und XI sind wie in Beispiel 1 vollständig klar und fallen durch Verdünnung mit Wasser als Präzipitiersysteme aus.

Aehnliche Ergebnisse wie in Beispiel 18 angegeben werden auch erzielt, wenn man in den Zusammensetzungen der Tabellen X und XI als Komponente (A) jeweils 5 Teile eines polymeren, quaternären Ammoniumsalzes eines Molekulargewichts von $1,5 \cdot 10^6$ bis $2,5 \cdot 10^6$, das 50 Mol% Strukturelemente der Formel (6), worin n zum Teil 1 und zum Teil 2 bedeutet, und 50 Mol% Strukturelemente der Formel (7) aufweist, oder 5 Teile eines handelsüblichen polymeren, quaternären Ammoniumsalzes, das Strukturelemente der Formel (12), worin n für 1 und $Q_2$ für Methyl und $Y^{\ominus}$ für das Methylsulfatanion stehen und Strukturelemente der Formel (7) (RETEN 210®) aufweist, einsetzt.

Vergleichsversuch I:

Man verfährt wie in Beispiel 1 angegeben, setzt jedoch in Abwesenheit eines amphoteren Tensids als Komponente ($B_3$) 130 Teile des nicht-ionogenen Tensids der Formel (24) als Komponente ($B_1$) in 520 Teilen entionisiertem Wasser und 5 Teile des anionischen Tensids der Formel (41) als Komponente ($B_2$) in 5 Teilen entionisiertem Wasser ein und verdünnt die Lösung D auf 1000 Teile mit ca. 238 Teilen entionisiertem Wasser.

Man erhält im Gegensatz zu den erfindungsgemässen vollständig klaren Zusammensetzungen gemäss einem der Beispiele 1 bis 38 eine opake bis cremeförmige Zusammensetzung, die zudem bei der Applikation auf Haar wesentlich schlechtere Ergebnisse ergibt, als dies z.B. für die Zusammensetzung gemäss Beispiel 1 der Fall ist.

Vergleichsversuch II:

Man verfährt wie in Beispiel 1 angegeben, setzt jedoch in Abwesenheit eines nicht-ionogenen Tensids als Komponente ($B_1$) 5 Teile des anionischen Tensids der Formel (41) als Komponente ($B_2$) in 5 Teilen entionisiertem Wasser und 130 Teilen des amphoteren Tensids der Formel (84) als Komponente ($B_3$) in 130 Teilen entionsiertem Wasser ein und verdünnt die Lösung D auf 1000 Teile mit ca. 628 Teilen entionisiertem Wasser.

Man erhält im Gegensatz zu den erfindungsgemässen vollständig klaren Zusammensetzungen gemäss einem der Beispiele 1 bis 38 eine opake bis cremeförmige Zusammensetzung, die zudem bei der Applikation auf Haar wesentlich schlechtere Ergebnisse ergibt als dies z.B. für die Zusammensetzung gemäss Beispiel 1 der Fall ist.

Patentansprüche

1. Als klare Formulierung vorliegende, wässrige Zusammensetzung aus polymeren Ammoniumsalzen und Tensiden, dadurch gekennzeichnet, dass sie mindestens
(A) ein in wässrigen Tensidsystemen lösliches oder mikroemulgierbares, polymeres, quaternäres Ammoniumsalz, das eine Molekulargewichtsverteilung von $10^3$ bis $10^9$ aufweist,
(B) ein ternäres Tensidsystem aus jeweils mindestens
($B_1$) einem nicht-ionogenen Tensid,
($B_2$) einem anionischen Tensid und
($B_3$) einem je eine positive Ladung und eine oder zwei negative Ladungen aufweisenden, amphoteren Tensid
enthält, wobei das anionische Tensid als Komponente ($B_2$) und das amphotere Tensid, sofern es zwei negative Ladungen als Komponente ($B_3$) aufweist, mindestens teilweise mit der Komponente (A) unter Ionenaustausch umgesetzt ist.

2. Zusammensetzung nach Anspruch 1, dadurch gekennzeichnet, dass sie als Komponente (A) quaternäre Stärke- oder Cellulose-Derivate, Poly(dialkylalkenylenammoniumhalogenid)-$\alpha,\omega$-(trialkanolammoniumhalogenid)-Verbindungen, Copolymerisate aus Dialkyldialkenylammoniumsalzen und Acrylamid oder Methacrylamid, Copolymerisate aus Acryl- oder Methacrylsäure, -amid oder -nitril und quaternären Ammoniumsalzen der Acrylsäurereihe, oder Homopolymerisate aus Dialkyldialkenylammoniumsalzen oder deren Gemische enthält.

3. Zusammensetzung nach Anspruch 1, dadurch gekennzeichnet, dass sie als nicht-ionisches Tensid für die Komponente ($B_1$) des ternären Tensidsystems alkoxylierte Fettalkohole, Fettsäuren, Fettsäureamide, Alkylphenole oder Kohlenhydrate, Glycosidäther oder Addukte aus Aethylen- und Propylenoxyd oder deren Gemische enthält.

4. Zusammensetzung nach Anspruch 1, dadurch gekennzeichnet, dass sie als anionisches Tensid für die Komponente ($B_2$) des ternären Tensidsystems ein Tensid der Formel $X_1^{\ominus}Z^{\oplus}$ enthält, worin $X_1^{\ominus}$ den Rest eines oberflächenaktiven Sarkosinats, Sulfats (Sulfatäthers), Sulfonats oder Sulfobernsteinsäurederivates oder deren Gemische und $Z^{\oplus}$ ein Alkalimetall- oder ein gegebenenfalls durch 1 bis 3 ($C_1$-$C_4$)-Alkyl- oder -Alkanolreste substituiertes Ammoniumkation bedeuten.

5. Zusammensetzung nach Anspruch 1, dadurch gekennzeichnet, dass sie als amphoteres Tensid für die Komponente ($B_3$) des ternären Tensidsystems ein intramolekular je eine positive und eine negative Ladung aufweisendes Betain oder Sulfobetain, das sich von einem aliphatischen Amin ableitet, oder ein je zwei negative Ladungen und eine positive Ladung aufweisendes, zwitterionisches Tensid der Formel $X_2^{\ominus}Z^{\oplus}$, worin $X_2^{\ominus}$ den zwei negative Ladungen aufweisenden Rest eines oberflächenaktiven N-Alkyl-iminodipropionats oder eines Dicarbonsäurederivats, das einen Glykokollrest enthält, bedeutet und $Z^{\oplus}$ die in Anspruch 4 angegebenen Bedeutungen hat, oder Gemische der genannten Tenside enthält.

6. Zusammensetzung nach einem der Ansprüche 1 bis 5, dadurch gekennzeichnet, dass sie 0,05 bis 2 Gewichtsprozent, bezogen auf Wirksubstanz, des Ammoniumsalzes als Komponente (A) und 4 bis 40 Gewichtsprozent, bezogen auf Wirksubstanz, des ternären Tensidsystems als Komponente (B) enthält.

7. Zusammensetzung nach Anspruch 6, dadurch gekennzeichnet, dass sie das nicht-ionogene Tensid ($B_1$), das anionische Tensid ($B_2$) und das amphotere Tensid ($B_3$) im ternären Tensidsystem (B) in einem Gewichtsverhältnis ($B_1$):($B_2$):($B_3$) von 1:(0,02 bis 6,0):(0,2 bis 4,0) enthält.

8. Zusammensetzung nach einem der Ansprüche 1 bis 7, dadurch gekennzeichnet, dass sie

0,1 bis 0,7 Gewichtsprozent der Komponente (A),
1 bis 10 Gewichtsprozent der Komponente ($B_1$),
0,1 bis 15 Gewichtsprozent der Komponente ($B_2$) und
1 bis 15 Gewichtsprozent der Komponente ($B_3$),

bezogen auf Wirksubstanz, enthält.

9. Zusammensetzung nach einem der Ansprüche 1 bis 8, dadurch gekennzeichnet, dass sie einen pH-Wert von 4 bis 8 aufweist.

10. Zusammensetzung nach einem der Ansprüche 1 bis 9, dadurch gekennzeichnet, dass sie zusätzlich zu den Komponenten (A), ($B_1$), ($B_2$) und ($B_3$) kosmetische Hilfsmittel als Komponente (C) enthält.

11. Zusammensetzung nach Anspruch 10, dadurch gekennzeichnet, dass sie 0,2 bis 5 Gewichtsprozent, bezogen auf Wirksubstanz, der Komponente (C) enthält.

12. Zusammensetzung nach einem der Ansprüche 1 bis 11, dadurch gekennzeichnet, dass sie als klare Formulierung vorliegt, die durch Zusatz von Wasser ausfällt.

13. Verfahren zur Herstellung der Zusammensetzung gemäss einem der Ansprüche 1 bis 12, dadurch gekennzeichnet, dass man zum amphoteren Tensid als Komponente ($B_3$) das quaternäre Ammoniumsalz als Komponente (A) gibt, wobei man gleichzeitig, sofern das amphotere Tensid eine positive Ladung und zwei negative Ladungen aufweist, das Tensid ($B_3$) mit dem Ammoniumsalz (A) unter Ionenaustausch mindestens teilweise

bei 10 bis 40°C umsetzt, anschliessend das nicht-ionogene Tensid als Komponente ($B_1$) und dann das anionische Tensid als Komponente ($B_2$) hinzugibt, wobei man gleichzeitig das Tensid ($B_2$) mit dem Ammoniumsalz (A) unter Ionenaustausch bei 10 bis 40°C mindestens teilweise umsetzt, und dann das Reaktionsgemisch mit kosmetischen Hilfsmitteln als fakultative Komponente (C) gegebenenfalls versetzt und schliesslich den pH-Wert der erhaltenen Zusammensetzung auf 4 bis 8 einstellt und die Zusammensetzung mit entionisiertem Wasser verdünnt.

14. Verwendung der Zusammensetzung gemäss einem der Ansprüche 1 bis 13 in haarkosmetischen Mitteln.

FO 7.1/PLP/rn*/mg*